# EUROPEAN PATENT SPECIFICATION

(11) **EP 4 185 883 B1**
(45) Date of publication and mention of the grant of the patent: **29.04.2026**
(21) Application number: 21790547.0
(22) Date of filing: 23.07.2021
(51) Int. Cl.: G01R 33/28, G01R 33/30, C07B 59/00, C07C 51/41, C07D 213/16, C07C 67/283, G01R 33/56

(54) **METHODS FOR GENERATION OF HYPERPOLARIZED COMPOUNDS USING PARAHYDROGEN**
VERFAHREN ZUR ERZEUGUNG VON HYPERPOLARISIERTEN VERBINDUNGEN UNTER VERWENDUNG VON PARAWASSERSTOFF
PROCÉDÉS POUR LA GÉNÉRATION DE COMPOSÉS HYPERPOLARISÉS À L'AIDE DE PARAHYDROGÈNE

(30) Priority: 23.07.2020 US 202063055367 P; 10.09.2020 US 202063076411 P
(43) Date of publication of application: 31.05.2023
(73) Proprietor: NVision Imaging Technologies GmbH, 89081 Ulm (DE)
(72) Inventor: SCHWARTZ, Ilai, 89231 Neu-Ulm (DE); KEIM, Michael, 89233 Neu-Ulm (DE)
(74) Representative: Huebner, Stefan Rolf
(86) International application number: PCT/IB2021/000493
(87) International publication number: WO 2022/018514

(56) References cited:
- US-A1- 2020 172 493
- FRANCESCA REINERI ET AL: "Use of Labile Precursors for the Generation of Hyperpolarized Molecules from Hydrogenation with Parahydrogen and Aqueous-Phase Extraction", ANGEWANDTE CHEMIE INTERNATIONAL EDITION, VERLAG CHEMIE, HOBOKEN, USA, vol. 50, no. 32, 22 June 2011 (2011-06-22), pages 7350 - 7353, XP072074968, ISSN: 1433-7851, DOI: 10.1002/ANIE.201101359
- RIPKA BARBARA ET AL: "Hyperpolarized fumarate via parahydrogen", CHEMICAL COMMUNICATIONS, vol. 54, no. 86, 9 October 2018 (2018-10-09), UK, pages 12246 - 12249, XP055868771, ISSN: 1359-7345, DOI: 10.1039/C8CC06636A
- EILLS JAMES ET AL: "Preservation of Nuclear Spin Order by Precipitation", CHEMPHYSCHEM, vol. 19, no. 1, 27 November 2017 (2017-11-27), DE, pages 40 - 44, XP055868747, ISSN: 1439-4235, Retrieved from the Internet <URL:https://api.wiley.com/onlinelibrary/tdm/v1/articles/10.1002%2Fcphc.201701189> [retrieved on 20211203], DOI: 10.1002/cphc.201701189
- TRUONG MILTON L. ET AL: "15 N Hyperpolarization by Reversible Exchange Using SABRE-SHEATH", THE JOURNAL OF PHYSICAL CHEMISTRY C, vol. 119, no. 16, 10 April 2015 (2015-04-10), US, pages 8786 - 8797, XP055792136, ISSN: 1932-7447, Retrieved from the Internet <URL:https://www.ncbi.nlm.nih.gov/pmc/articles/PMC4419867/pdf/jp5b01799.pdf> [retrieved on 20211203], DOI: 10.1021/acs.jpcc.5b01799

## Description

### CROSS-REFERENCE TO RELATED APPLICATIONS

### TECHNICAL FIELD

The disclosed embodiments generally relate to generation of hyperpolarized materials for use in nuclear magnetic resonance, magnetic resonance imaging, or similar applications.

### BACKGROUND

Parahydrogen induced polarization (PHIP) is a method for polarizing metabolites for hyperpolarized (HP) Magnetic Resonance Imaging (MRI), with low cost and high throughput. Parahydrogen induced polarization (PHIP), parahydrogen induced polarization with sidearm hydrogenation (PHIP-SAH), and signal amplification by reversible exchanges (SABRE) can be used to polarize metabolites (e.g. fumarate or pyruvate). However, existing polarization approaches may be unsuitable for preclinical or clinical hyperpolarization MRI applications, as such approaches may be unable to achieve a sufficient sample volume, purity, polarization, or concentration.
Ripka, Barbara et al in "Hyperpolarized fumarate via parahydrogen", Chem Commun, 2018, vol 54, no 86, pages 12246 ff disclose a method of producing hyperpolarized [1-¹³C]fumarate in the proton nuclear spin singlet state by pairweise trans-addition of parahydrogen to a acetylene dicarboxylate precursor using a ruthenium-based catalyst in water.
Eills, James et al in "Preservation of Nuclear Spin Order by Precipitation", ChemPhysChem, vol 19, no 1, pages 40 ff, report that a non-equilibrium nuclear spin order of ¹³C- and ²H-labeled sodium fumarate can survive precipitation from aqueous solution and redissolution, with precipitation and dissolution achieved by altering the pH.
Reineri, Francesca et al in "Use of Labile Precursors for the Generation of Hyperpolarized Molecules from Hydrogenation with Parahydrogen and Aqueous Phase Extraction" discloses a method of obtaining hyperpolarised succinate that comprises hyperpolarising maleic anhydride in an organic solvent to produce succinic anhydride as an intermediate precursor product, and obtaining succinate by subsequent aqueous phase extraction.

### SUMMARY

Methods of generating a solution comprising a hyperpolarized target compound with the features of claim 1 and, alternatively, with the features of claim 8 provided. Disclosed systems and methods relate to generation of hyperpolarized target compounds using at least one of PHIP, PHIP-SAH, or SABRE. Generation of the hyperpolarized materials can include application of a sequence of microwave pulses or modulation of a magnetic field. According to the invention, target compound is hyperpolarized in a solution and then induced to precipitate out of that solution. The precipitate is redissolved in a specified volume of solvent to form a solution having a desired concentration of the hyperpolarized target compound.

Disclosed embodiments include a method of generating a solution comprising a hyperpolarized target compound. The method includes generating a first solution comprising an organic solvent, having a first concentration of a precursor of a target compound, by inducing a hydrogenation reaction between parahydrogen and the precursor using parahydrogen induced polarization (PHIP), thereby creating a population difference in proton spins in the parahydrogenated precursor. The method further includes applying a polarization transferring waveform to the first solution, the polarization transferring waveform configured to transfer the created population difference to polarization on a target nuclear spin of the parahydrogenated precursor. The method further includes precipitating a first amount of the target compound from the first solution. The method further includes separating the precipitated first amount from the first solution. The method also includes generating a second solution having a second concentration of the target compound by combining the precipitated first amount with a solvent.

Disclosed embodiments also include a method of generating a solution comprising a hyperpolarized target compound. The method includes generating a first solution including an organic solvent, a target compound, parahydrogen, and a catalyst, the catalyst configured to bind the parahydrogen and the target compound. The method includes transferring spin order from the parahydrogen to polarization on the target compound via the catalyst using the SABRE effect. The method includes generating a second solution including the target compound and the catalyst, the generation comprising: (a) generating a precipitate of the target compound; (b) separating the precipitate from the first solution; and (c) combining at least some of the precipitate with a solvent, thereby generating the solution comprising the hyperpolarized target compound.

Disclosed embodiments also include a system for generating a polarized solution including a hyperpolarized target compound. The system can include a first sub-system for generating a hyperpolarized precipitate and a second sub-system for dissolution of a precipitated fraction. The first sub-system can include a hydrogenation device, a polarization device, a waveform generator, and a separation system. The hydrogenation device can be configured to generate a first solution by combining parahydrogen gas, a solvent, and a precursor or a target compound. The polarization device can be configured to receive the first solution and generate a second solution. The polarization device can include a polarization chamber having a volume of at least 1 ml. The waveform generator can be coupled to the polarization device and can be configured to provide a polarization transfer signal. The separation system can be configured to separate a precipitated fraction of the hyperpolarized target compound from the second solution. The second sub-system for dissolution of the precipitated fraction can include a dissolution chamber configured to contain the precipitated fraction and to receive a second solvent for dissolving the precipitated fraction.

Disclosed embodiments also include a method. The method can include operations of modulating an amplitude of a magnetic field applied to a sample contained within a magnetic shield, a duration of the modulation being greater than 100 milliseconds and less than 20,000 milliseconds, the amplitude of the magnetic field during modulation being less than 2 microteslas, and a spatial deviation of the magnetic field during the modulation being less than 0.5 microteslas over a volume of the sample greater than 10 milliliters and less than 2,000 milliliters. The modulation can be configured to transfer a population difference in parahydrogenated proton spin states to polarization on a target nuclear spin of a parahydrogenated target compound in the sample.

Disclosed embodiments also include system. The system can include a magnetic shield, a sample reservoir disposed within the magnetic shield, and a magnetic field coil disposed within the magnetic shield, electrically connected to a signal generator, and configured to generate, when driven by the signal generator, a magnetic field, a spatial deviation of the magnetic field being, when an amplitude of the magnetic field is less than 2 uT, less than 0.5 uT over a volume of the sample reservoir, the volume of the sample reservoir being greater than 10 ml and less than 2,000 ml. The system can further include at least one computer-readable media containing instructions that, when executed by the at least one processor of the system, cause the system to perform operations. The operations can include modulating the amplitude of the magnetic field, a duration of the modulation being greater than 100 milliseconds and less than 20,000 milliseconds, the amplitude of the magnetic field during modulation being less than 2uT. The modulation can be configured to transfer a population difference in parahydrogenated proton spin states to polarization on a target nuclear spin of a target compound in the sample contained in the sample reservoir.

It is to be understood that both the foregoing general description and the following detailed description are exemplary and explanatory only and are not restrictive of the disclosed embodiments, as claimed.

### BRIEF DESCRIPTION OF THE DRAWINGS

The accompanying drawings, which comprise a part of this specification, illustrate several embodiments and, together with the description, serve to explain the principles and features of the disclosed embodiments. In the drawings:
**FIG. 1** illustrates an exemplary process 100 for PHIP polarization, consistent with disclosed embodiments.
**FIG. 2** depicts an exemplary schematic of a polarizer 200 for obtaining a high-concentration, biocompatible solution with hyperpolarized target compounds, consistent with disclosed embodiments.
**FIGs. 3** and **4** depict embodiments of large-scale polarizers, consistent with disclosed embodiments.
**FIGs. 5A** and **5B** demonstrate the purity of a pyruvate precursor synthesized in accordance with disclosed embodiments.
**FIG. 6A** depicts the ¹³C NMR spectrum of hyperpolarized E-cinnamyl pyruvate-1-¹³C, demonstrating polarization transfer, in accordance with the disclosed embodiments.
**FIG. 6B** depicts the ¹H NMR spectrum of hydrogenated (3-phenylpropargyl)pyruvate-1-¹³C, the successful hydrogenation of a (3-phenylpropargyl)pyruvate-1-¹³C precursor, in accordance with disclosed embodiments.
**FIG. 7A** shows separation of a sodium pyruvate precipitate from a solution using a frit containing the filter, in accordance with disclosed embodiments.
**FIG. 7B** shows a scanning electron microscope (SEM) image of precipitated and washed sodium pyruvate resulting from the cleavage of cinnamyl pyruvate with sodium hydroxide solution, in accordance with disclosed embodiments.
**FIG. 8** depicts the ¹H NMR spectrum of precipitated and washed sodium pyruvate resulting from the cleavage of cinnamyl pyruvate with sodium hydroxide solution, after redissolution in D₂O (400.13 MHz, D₂O), in accordance with disclosed embodiments.
**FIG. 9A** depicts an experimental setup for generating a hyperpolarized target compound, consistent with disclosed embodiments.
**FIG. 9B** depicts the profile of a magnetic field sweep in the magnetic shield of the experimental setup of **FIG. 9A****,** in accordance with disclosed embodiments.
**FIG. 9C** shows initial results of the hyperpolarization in sodium fumarate achieved before and after precipitation and redissolution of the sodium fumarate, in accordance with disclosed embodiments.
**FIG. 10** depicts the results of cytotoxicity experiments performed using samples of precipitated, washed and redissolved sodium fumarate generated in accordance with disclosed embodiments

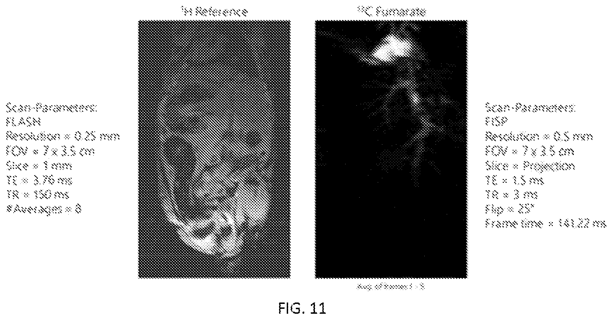

**FIG. 11** depicts images and scan parameters for preclinical MRI images obtained in a mouse using precipitated, washed and redissolved samples of hyperpolarized sodium fumarate generated in accordance with disclosed embodiments.
**FIG. 12A** depicts the ¹³C NMR spectrum of hyperpolarized sodium fumarate-1-¹³C (80 MHz, D₂O) generated in accordance with disclosed embodiments.
**FIG. 12B** shows the large volume hyperpolarized sample from which a small amount was placed into an NMR vial and measured as shown in **FIG. 12A****.**
**FIG. 13** depicts a dry-shipper including a permanent magnet insert suitable for transporting the hyperpolarized precipitated particles before redissolution, in accordance with disclosed embodiments.

### DETAILED DESCRIPTION

Reference will now be made in detail to exemplary embodiments, discussed with regard to the accompanying drawings. In some instances, the same reference numbers will be used throughout the drawings and the following description to refer to the same or like parts. Unless otherwise defined, technical or scientific terms have the meaning commonly understood by one of ordinary skill in the art. The disclosed embodiments are described in sufficient detail to enable those skilled in the art to practice the disclosed embodiments. It is to be understood that other embodiments may be utilized and that changes may be made without departing from the scope of the invention as defined by the appended claims. Thus, the materials, methods, and examples are illustrative only and are not intended to be necessarily limiting.

Recent work in the field of nuclear magnetic resonance (NMR) and magnetic resonance imaging (MRI) has demonstrated that NMR and MRI signals associated with a variety of biorelevant imaging agents can be enhanced by many orders of magnitude using a variety of so-called hyperpolarization techniques. Such drastic signal enhancement allows spectroscopic analysis of the biorelevant imaging agent as it is metabolized by various tissues at different locations within a body. Analysis of the metabolic information determined by such spectroscopic imaging may allow non-invasive determination of a health state of tissue within a body. For example, abnormal metabolism of the biorelevant imaging agent may be indicative of a disease such as cancer at some location in the body.

Existing techniques for hyperpolarizing biorelevant imaging agents include dissolution dynamic nuclear polarization (DNP), parahydrogen induced polarization (PHIP), PHIP-sidearm hydrogenation (PHIP-SAH), and signal amplification by reversible exchange (SABRE). However, existing techniques cannot produce clinically relevant volumes and concentrations of biorelevant imaging agents having sufficient polarization and purity. Accordingly, there is a need for hyperpolarization methods and systems that produce hyperpolarized biorelevant imaging agents at clinically relevant concentrations.

The disclosed embodiments include systems and methods for producing target compounds, such as biorelevant imaging agents, in clinically relevant polarizations, concentrations, volumes and purity. Disclosed embodiments provide technical improvements in polarizing target compounds in solution. These technical improvements support increases in target compound concentration, the degree of target compound polarization, and increased solution volumes. A magnetic shield can be used to create a large-volume, homogenous, low-amplitude magnetic field. The magnetic field can be modulated to polarize a target compound in a solution contained within the magnetic shield. Accordingly, a greater amount of a more-highly polarized target compound can be produced.

Disclosed embodiments also provide technical improvements in the separation of hyperpolarized target compounds from a solution. The target compounds can be induced to precipitate from the solution, enabling purification of the target compound and redissolution of the target compounds into another solution. Thus, the characteristics (e.g., volume, concentration, solute, presence of additives) of the solution used for polarization of the target molecule can be at least partially separated from the characteristics of the second solution used for clinical applications.

The disclosed embodiments can be used together, or can be used separately. For example, the disclosed technical improvements in polarizing target compounds can be used with the disclosed improved separation methods, or other separation methods. Likewise, the disclosed technical improvements in separating target compounds can be used with the disclosed improved polarization methods, or other polarization methods.

Hyperpolarization can be a condition in which an absolute value of a difference between a population of spin states (e.g., nuclear spin states, proton spin states, or the like) being in one state (e.g., spin up) and a population of a spin states being in another state (e.g., spin down) exceeds the absolute value of such a difference at thermal equilibrium.

Parahydrogen can be used as a source of polarization, consistent with disclosed embodiments. Parahydrogen, as described herein, is a form of molecular hydrogen in which the two protons spins are in the singlet state. The disclosed embodiments are not limited to a particular method of generating parahydrogen. Parahydrogen may be formed in a gas form or in a liquid form. Parahydrogen may be generated in gas form by flowing hydrogen gas at low temperature through a chamber with a catalyst (e.g., iron oxide or another suitable catalyst). The hydrogen gas can contain both parahydrogen and orthohydrogen. The low temperature can bring the hydrogen gas to thermodynamic equilibrium in the chamber, increasing the population of parahydrogen. The disclosed embodiments are not limited to a particular parahydrogen generation location. Parahydrogen can be generated at a first location and subsequently transported to a second location for use. In some embodiments, a first location may be a chamber, which may be part of a container, bottle, holder or other regions capable of holding a gas or a liquid. Such a chamber may be maintained at a suitable pressure or temperature. In some embodiments, the first location may refer to a physical location such as a room, a lab, a particular warehouse, hospital or other location where the parahydrogen may be generated. The disclosed embodiments are not limited to a particular parahydrogen transport method. The generated parahydrogen may be transported in a chamber, which may be different from the chamber where the parahydrogen was generated. The chamber transporting the parahydrogen gas may be maintained at a suitable pressure or temperature, which may be transported by vehicle or persons. Transporting the parahydrogen may involve moving the parahydrogen from one container to a different container. Transporting the parahydrogen may involve moving the parahydrogen within the same location, such as from one part of a room to another part of the room. Transporting the parahydrogen may involve moving the parahydrogen from one room in a building to a different room in the same building or to a nearby building. Transporting the parahydrogen may involve moving the parahydrogen to a different location in another part of the same city, or a different city. Transporting the parahydrogen may involve bringing the parahydrogen to a vicinity of a polarizer or an NMR/MRI device. Transporting the parahydrogen may involve packaging or shipping the parahydrogen in suitable containers.

A population difference between spin states can be the difference between the population of the two spin states divided by the total population of these two spin states. A population difference may be expressed as a fractional population difference or a percentage population difference. In certain embodiments, the fractional population difference is at least about 0.1, 0.2, 0.3, 0.4, 0.5, 0.6, 0.7, 0.8, 0.9, or more. In certain embodiments, the fractional population difference is at most about 0.9, 0.8, 0.7, 0.6, 0.5, 0.4, 0.3, 0.2, 0.1, or less. In certain embodiments, the fractional population difference is within a range defined by any two of the preceding values.

Hydrogen gas can exhibit a population difference between proton spin states greatly exceeding the population difference between proton spin states at thermal equilibrium. Parahydrogen can have a large population difference between the singlet spin state and any of the triplet spin states. In the case of Iz1Iz2, there is a large population difference for example between the spin state |↑>|↓> and the spin state |↑>|↓>. The population difference in proton spin states can be more than about 0.1 (e.g., a 10% difference in spin states - 55 % of the parahydrogen molecules in a sample being in the singlet state and 45% in the triplet state), 0.2, 0.3, 0.4, 0.5, 0.6, 0.7, 0.8, 0.9, or more; or can be less than about 0.9, 0.8, 0.7, 0.6, 0.5, 0.4, 0.3, 0.2, 0.1, or less.

Target compounds can include materials suitable for use in NMR or MRI operations (e.g., "NMR materials"). In some embodiments, such an NMR material may increase NMR/MRI signal and signal-to-noise ratio (SNR). In some embodiments, the NMR material can be suitable for use in solution NMR spectroscopy. In some embodiments, the NMR material may be a metabolite (e.g., a molecule with a biological relevance such as an amino acid, a saccharide, a derivative thereof, or the like), such as a metabolite suitable for use in an NMR metabolomics application. In some embodiments, the NMR material may be suitable for *in-vitro* probing of the metabolism of a cell culture or other biological tissue. In some embodiments, the NMR material may be used in an NMR probe to investigate a transient effect in which high signal enhancement due to hyperpolarization is needed, such as proton exchange between water and biomolecules. In some embodiments, the NMR material can be a small molecule or metabolite suitable for injection into a cell, tissue or organism for detection in an MRI scan. In some embodiments, the NMR material can be introduced into a chamber for further analysis by NMR or MRI operations. In some embodiments, the NMR material can be enriched with ¹³C atom(s).

Consistent with disclosed embodiments, the target compound can be pyruvate, lactate, bicarbonate, fumarate, urea, alpha-ketoglutarate, dehydroascorbate, glutamate, glutamine, acetate, dihydroxyacetone, acetoacetate, glucose, ascorbate, zymonate, imidazole, nicotinamide, nitroimidazole, pyrazinamide, isoniazid, a conjugate acid of any of the foregoing, natural and unnatural amino acids, esters thereof, or ¹³C or ¹⁵N enriched versions of any of the foregoing.

Consistent with disclosed embodiments, a target compound can be generated from a "precursor" of the target compound. Such generation can include one or more chemical reactions. In some embodiments, the target compound can be generated from the precursor by hydrogeneration of one or more unsaturated bonds (e.g., unsaturated carbon-carbon bonds) of the precursor. For example, when the target compound is fumarate, one or more unsaturated bonds of acetylenedicarboxylic acid can be hydrogenated to generate fumaric acid. In some embodiments, the target compound can be generated from the precursor by cleaving from the precursor a sidearm to yield the target compound. In some embodiments, the precursor can be an ester, with a generic formula of -COOR. Accordingly, in such embodiments, the term "sidearm" may refer to the R moiety of the ester. In some embodiments, the sidearm may include the substituents and functional groups directly bound to the ester oxygen bound by carbons on both sides. For example, the precursor can be an enol or ynol ester of the target compound, wherein the specific enol or ynol may be referred to as the sidearm. As a further example, the target compound can be pyruvate. When the precursor is vinyl pyruvate and the sidearm is a vinyl functional group. When the precursor is cinnamyl pyruvate, the sidearm is a phenyl allyl functional group. When the precursor is allyl pyruvate, the sidearm is an allyl functional group. When the precursor is propargyl pyruvate, the sidearm is a propargyl functional group. In some embodiments, the target compound can be generated from the precursor by hydrogeneration and cleaving of a sidearm. For example, the precursor can be hydrogenated and the sidearm subsequently cleaved to yield the target compound. The hydrogenation may occur at a location on the sidearm or the target compound.

Consistent with disclosed embodiments, hydrogenation can be performed using parahydrogen (e.g., parahydrogenation). Such parahydrogenation can create a population difference in proton spins in the parahydrogenated precursor. Consistent with disclosed embodiments, the population difference in proton spins can be transferred to polarization on a target nuclear spin of the parahydrogenated precursor, creating a hyperpolarized parahydrogenated precursor. When the target compound is generated from the precursor by hydrogenation, the hyperpolarized parahydrogenated precursor can be the hyperpolarized target compound. When the target compound is generated from the precursor by hydrogenation and cleaving of a sidearm, the hyperpolarized parahydrogenated precursor can be cleaved to yield the hyperpolarized target compound.

Various embodiments of the present disclosure disclose a method for preparing hyperpolarized target compounds. In some embodiments, the target compound can be generated from the precursor through parahydrogenation and polarization using PHIP or SABRE, while in other embodiments the target compound can be generated from the precursor through parahydrogenation and polarization using PHIP-SAH, followed by cleavage of a sidearm.

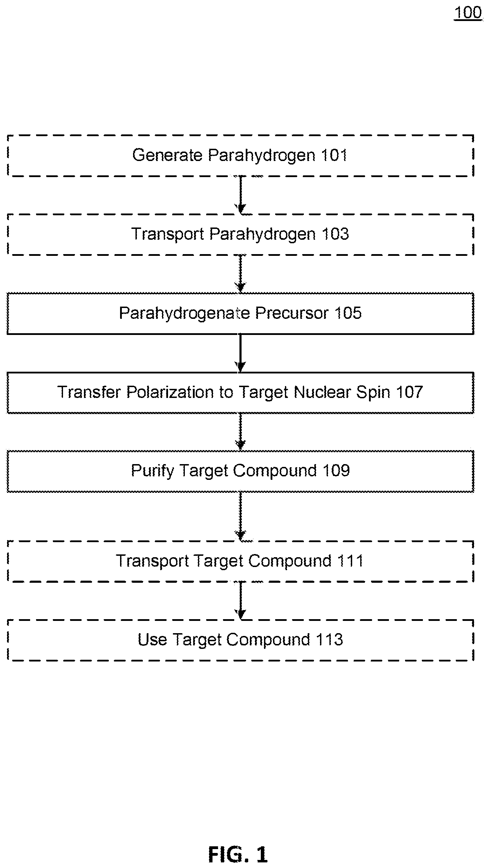

**FIG. 1** illustrates an exemplary process 100 for PHIP polarization, consistent with disclosed embodiments. Process 100 can be used for large-scale PHIP polarization to generate target compounds for in vitro or in vivo MRI. A similar process can be used for SABRE polarization, with the hydrogenation in step 105 replaced with an exchangeable binding of the target compound and parahydrogen to a polarization catalyst. This depiction of process 100 is not intended to be limiting. Envisioned embodiments can include additional steps, or fewer steps; or steps could be combined or divided. For example, process 100 could begin with step 105, using previously generated or obtained parahydrogen. As an additional example, process 100 could terminate with purification or separation of the target compound in step 109. Likewise, the transportation in step 103 and step 111 may be optional. Furthermore, while depicted as being used in an MRI, the target compound is not necessarily limited to such a use.

Optional step 101 of process 100 depicts generation of parahydrogen, consistent with disclosed embodiments. Parahydrogen can be generated by flowing cold hydrogen gas through a chamber with a catalyst such as iron oxide, bringing the parahydrogen and orthohydrogen to their thermodynamic equilibrium. In some embodiments, at low temperatures, parahydrogen may be increasingly populated. In some alternative embodiments, liquid parahydrogen may be prepared similarly. Parahydrogen can be generated in advance, or as needed, consistent with disclosed embodiments.

Optional step 103 of process 100 depicts transport of pressurized parahydrogen containers, for example gas bottles, consistent with disclosed embodiments. These parahydrogen containers may be filled and transported to the vicinity of MRI scanners. In some alternative embodiments, the pressurized parahydrogen may be liquid parahydrogen bottles, which may also be filled and shipped. In an alternate embodiment, the parahydrogen generator is connected to a PHIP polarizer, therefore relieving the need for parahydrogen gas containers.

Step 105 of process 100 depicts parahydrogenation of a precursor, consistent with disclosed embodiments. In some embodiments, parahydrogen gas may combined with the precursor (e.g., in a solution or mixture), hydrogenating the precursor and creating Iz1Iz2, the lower energy state between |↑>|↓>, |↑>|↓> or singlet spin order on two hydrogens spins.

Step 107 depicts a transfer of a population difference in parahydrogenated proton spin states to polarization on a target nuclear spin of the parahydrogenated precursor, consistent with disclosed embodiments. Such transfer can be accomplished, consistent with disclosed embodiments, using at least one of modulation of a magnetic field applied to a solution containing the parahydrogenated precursor or application of a sequence of radiofrequency pulses. Such transfer can generate a hyperpolarized precursor. In some embodiments, the precursor to be hydrogenated and hyperpolarized may include a sidearm (e.g., the precursor can be an ester of a target compound). The hydrogenation reaction can occur in the sidearm, and spin order may be transferred to polarization on a target nuclear spin at a location in the target compound.

Step 109 depicts the purification or separation from the original solution of the hyperpolarized precursor (or a hyperpolarized target compound generated from the hyperpolarized precursor), consistent with disclosed embodiments. In some embodiments using PHIP-SAH, the purification or separation can include cleaving the hyperpolarized precursor to generate the hyperpolarized target compound. In embodiments using PHIP or SABRE, the hyperpolarized precursor can comprise the hyperpolarized target compound. The purification or separation can include inducing precipitation of the hyperpolarized precursor or hyperpolarized target compound from the original solution.

Optional step 111 depicts transport of the precipitate, consistent with disclosed embodiments. The precipitate can be transported in a transport device with a magnetic field and optionally a coolant.

Optional step 113 depicts use of the target compound, consistent with disclosed embodiments. In some embodiments, upon redissolution of the precipitated particles (and optionally after regulating temperature and pH: cleavage of the precursor and separating of the target compound; or any additional chemical reactions) the hyperpolarized target compound may be injected into a patient for use in MRI imaging (e.g., a hyperpolarized MRI experiment, or another suitable imaging action).

### Target Compounds

In some embodiments, the target compound may be chosen such that following the hydrogenation and other potential chemical reactions, one of the products is a hyperpolarized target compound, such as a hyperpolarized biorelevant imaging agent, usable in HP MRI applications. In some embodiments, the hyperpolarized target compound can be produced through additional chemical reactions. Such additional chemical reactions may include adding a sidearm containing an unsaturated moiety for hydrogenation and polarization to transfer polarization to the target compound, followed by cleavage of the sidearm, e.g., by hydrolysis. For example, the ester of the target compound may be used for polarization using the PHIP-SAH method. In the PHIP-SAH method, hydrogenation by parahydrogen is conducted on a precursor, which is a chemical derivative of a target compound, such as a biorelevant imaging agent. The chemical derivative generally comprises an ester containing the biorelevant imaging agent and a sidearm containing an unsaturated carbon-carbon bond. Parahydrogen is used to hydrogenate the unsaturated carbon-carbon bond and transfer spin order from parahydrogen to the target compound. Following hydrogenation and polarization transfer, the ester may be cleaved to produce the hyperpolarized target compound. In some embodiments, various precursors and esters of the target compound may be used. In some embodiments the esters are enol or ynol esters of a carboxylic acid, in other embodiments they are esters such as allyl and propargyl esters. For example, when pyruvate is the target compound, precursors and esters of the target compound may be enol or ynol esters of pyruvate such as styryl pyruvate (IIIa) or phenylethynyl pyruvate (IVa).

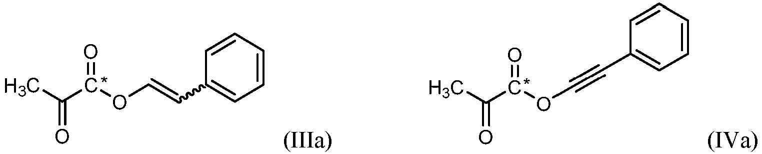

Various embodiments for the present disclosure disclose transferring the spin order from the ¹H spins in the sidearm to a nuclear spin of the sidearm or the target compound itself. In some embodiments, polarization may be hyperpolarization (HP), which refers to an excess population in one or more nuclear spin states compared to a thermal equilibrium nuclear spin state distribution in a given magnetic field. Such thermal equilibrium distribution may be described by the Boltzmann distribution.

In some embodiments, spin order can be transferred first from spin order on parahydrogen to spin order or polarization on a first nuclear spin in the sidearm, and from there to a second nuclear spin in the target compound. For example, the first nuclear spin could be a ¹H nuclear spin in the sidearm, and the second nuclear spin could be a ¹³C nuclear spin in the target compound, or alternatively a ¹³C or ¹⁵N spin in the sidearm followed by transfer to a ¹³C or ¹⁵N spin in the target compound. Interestingly, as ¹³C-¹³C J couplings can be still larger than 1 Hz even for a three bond distance, in a preferred embodiment a ¹³C nuclear spin further in the target compound can be hyperpolarized. For example, this enables the polarization of [2-¹³C] pyruvate by transfer from a ¹³C spin in the sidearm.

Various embodiments of the present disclosure disclose hydrogenating a sidearm of the target compound using the parahydrogen.

In some embodiments, the precursor may be chosen such that following the hydrogenation and other potential chemical reactions, one of the products is a target compound usable in HP MRI applications. In some embodiments, the target compound can be produced through additional chemical reactions following hydrogenation. Such additional chemical reactions may include adding a sidearm containing an unsaturated moiety for hydrogenation and polarization, in order to transfer polarization to the target compound, followed by cleavage of the sidearm of the target compound, e.g., by hydrolysis. For example, the ester of the target compound may be used for polarization using the PHIP-SAH method. Following hydrogenation and polarization transfer, the ester may be hydrolyzed to produce the hyperpolarized target compound.

In this invention, hydrolysis is defined as the cleavage of a molecule via a nucleophilic substitution reaction, with the addition of the elements of water. It can be also performed under anhydrous conditions under the presence of hydroxide ions.

### Parahydrogenation

Consistent with disclosed embodiments, a precursor to the target compound can be parahydrogenated by combining the precursor, parahydrogen, and a hydrogenation catalyst. The disclosed embodiments are not limited to a particular method of generating a parahydrogenated precursor. In some embodiments, the precursor can be added to a mixture containing parahydrogen. In some embodiments, parahydrogen gas can be added to a solution containing the precursor (e.g., the parahydrogen gas can be bubbled into such a solution). In hydrogenating the precursor, the parahydrogen can create Iz1Iz2 order, preferential population of the lower energy state between |↑>|↓>, |↓>|↑> or singlet spin order on two hydrogens spins in the precursor.

The precursor can have an unsaturated bond that can be hydrogenated by the parahydrogen gas. Following combination of the precursor and the parahydrogen, more than about 50%, more than about 60%, more than about 70%, more than about 80%, more than about 90%, or almost all of precursor may be hydrogenated. Similarly, less than about 90%, less than about 80%, less than about 70%, less than about 60%, less than about 50%, or fewer of the precursor may be hydrogenated. Or the percentage of hydrogenation can fall within a range defined by any two of the preceding values.

In some embodiments, the parahydrogenated precursor can have at least a 10%, 20%, 40% population difference in the parahydrogenated proton spin states. In some embodiments, the population difference is between spin states which include the parahydrogenated protons as well as other nuclear spins, for example additional protons on the compound. In some embodiments, the parahydrogenated precursor can include a sidearm and the parahydrogenated spins can be located on the sidearm.

In some embodiments, a concentration of the hydrogenation catalyst during hydrogenation can be at most about 50 mM, 40 mM, 30 mM, 20 mM, 10 mM, 9 mM, 8 mM, 7 mM, 6 mM, 5 mM, 4 mM, 3 mM, 2 mM, 1 mM, 0.9 mM, 0.8 mM, 0.7 mM, 0.6 mM, 0.5 mM, 0.4 mM, 0.3 mM, 0.2 mM, 0.1 mM, or less; at least about 0.1 mM, 0.2 mM, 0.3 mM, 0.4 mM, 0.5 mM, 0.6 mM, 0.7 mM, 0.8 mM, 0.9 mM, 1 mM, 2 mM, 3 mM, 4 mM, 5 mM, 6 mM, 7 mM, 8 mM, 9 mM, 10 mM, 20 mM, 30 mM, 40 mM, 50 mM, or more; or within a range defined by any two of the preceding values.

The disclosed embodiments can include methods implemented by disclosed systems for generating a hyperpolarized target compound. The disclosed methods can include mixing, by a mixing mechanism, a solution including a precursor to the target compound and a hydrogenation catalyst. A mixing mechanism may be a device for introducing, holding, and facilitating a blend, mixture, or solution of two or more materials. In some embodiments, the mixing mechanism may be disposed in a chamber, and the mixing may occur inside the chamber. In some embodiments, the solution may be mixed at a location away from the chamber. The solution may be at least about 1 ml, 2 ml, 3 ml, 4 ml, 5 ml, 6 ml, 7 ml, 8 ml, 9 ml, 10 ml, 20 ml, 30 ml, 40 ml, 50 ml, 60 ml, 70 ml, 80 ml, 90 ml, 100 ml, or more in volume; or at most 100 ml, 90 ml, 80 ml, 70 ml, 60 ml, 50 ml, 40 ml, 30 ml, 20 ml, 10 ml, 9 ml, 8 ml, 7 ml, 6 ml, 5 ml, 4 ml, 3 ml, 2 ml, 1 ml or less in volume; or within a volume range defined by any two of the preceding values.

In some embodiments, the mixing mechanism may be a gas-liquid exchange mechanism. For example, the gas-liquid exchange mechanism may be a bubbler or a diffusion system. In some embodiments, the mixing mechanism may comprise membranes adapted to permit diffusion of molecular hydrogen. In some embodiments the mixing can be performed using a spray chamber, where the solution is sprayed into a chamber filled with pressurized parahydrogen.

In some embodiments, the catalyst can be any molecule, complex or particle system that catalyzes hydrogenation. In some embodiment, there may be provided a homogeneous metal catalyst such as a rhodium complex or a ruthenium complex. The rhodium complex can be used for coordination and activation of precursor molecules and parahydrogen. In some embodiments, a heterogeneous metal catalyst connected to a nanoparticle can be used.

Various embodiments of the present disclosure disclose introducing the solution to a chamber configured to hold the solution during polarization transfer. In some embodiments, the solution may be mixed in the chamber. In some embodiments, the solution may be hydrogenated in the chamber. In some embodiments, the chamber can be within a magnetic shield (e.g., a mu metal shield). The magnetic shield can reduce the effect of the Earth's magnetic field (or other extraneous magnetic fields), permitting modulation of the amplitude of a low-level magnetic field applied to the solution. Accordingly, placing the solution within the chamber can include placing the solution within the magnetic shield.

As described herein, in some embodiments parahydrogenation can occur prior to polarization transfer (e.g., prior to the modulation of the amplitude the magnetic field applied to the solution, or the like). In various embodiments, parahydrogenation can occur during polarization transfer. For example, parahydrogen can be combined with (e.g., flowed or bubbled through the solution) the solution during modulation of the amplitude of the magnetic field.

In certain embodiments, the parahydrogen gas can be combined with the solution in the hydrogenation chamber at pressure. The pressure can be at least about 10 bar, 15 bar, 20 bar, 30 bar, 50 bar, or more; the pressure can be at most about 50 bar, 30 bar, 20 bar, 15 bar, 10 bar or less (1bar = 10⁵ Pa); or the pressure can be within a range defined by any two of the preceding values. In such embodiments, the parahydrogen can be combined with the solution in a metallic chamber capable of withstanding the pressure. The parahydrogen can be combined with the solution for (or the dissolution of the parahydrogen can occur in less than) a time interval. The time interval can be less than about 90 seconds, 60 seconds, 30 seconds, 20 seconds, 10 seconds, 5 seconds, 3 seconds or less; the time interval can be more than about 3 seconds, 5 seconds, 10 seconds, 20 seconds, 30 seconds, 60 seconds, 90 seconds, or more; or the time interval can be within a range defined by any two of the preceding values.

The envisioned systems and methods can be used with any suitable polarization transfer scheme that uses parahydrogen to induce polarization (e.g., PHIP, PHIP-SAH, SABRE, or any other suitable polarization transfer method).

When used with SABRE, the parahydrogen molecule and the target compound can both be bound to a polarization transfer catalyst. Polarization transfer can then occur, at least in part, while parahydrogen and the target compound are bound. In some embodiments, parahydrogen gas is dissolved in a first solution including the target compound and the polarization transfer catalyst. The first solution can be any solvent which enables SABRE polarization, including aqueous solutions (outside the scope of the invention) or, in accordance with the invention, organic solvents, e.g. deuterated chloroform, deuterated acetone, deuterated ethanol or deuterated methanol.

In such embodiments, the polarization transfer catalyst can reversibly bind the target compound (e.g. pyridine or another suitable target compound) and parahydrogen, enabling spin order or polarization transfer to the target compound. Following such transfer, the average polarization of the compounds is at least about 1%, 10%, 30%, 50%; at most about 90%, 50%, 30%, 10%, or 1%; or within a range defined by any two of the preceding values. The polarized nuclear species can be ¹⁵N, ¹³C, ¹H, ³¹P, ¹⁹F or other nuclear species that can be polarized by SABRE.

In some embodiments, the polarization transfer catalyst can be or include a metallic complex, for example an iridium organometallic complex (e.g., the iridium organometallic complex [IrCl(COD)(1,3-bis(2,4,6-trimethylphenyl)imidazol-2-ylidene)]). In some embodiments, the polarization transfer catalyst can be or include a heterogeneous metal catalyst connected to a nanoparticle.

### Polarization Transfer

In certain embodiments, the concentration of the precursor (in embodiments using PHIP-SAH) or target compound (in embodiments using PHIP or SABRE) in the solution prior to polarization transfer can be more than about 10 mM, 20 mM, 30 mM, 40 mM, 50 mM, 60 mM, 70 mM, 80 mM, 90 mM, 100 mM, 250 mM, 400 mM, 600 mM, 1000 mM, or more. In certain embodiments, the concentration of the precursor can be less than about 1000 mM, 600 mM, 400 mM, 250 mM, 100 mM, 90 mM, 80 mM, 70 mM, 60 mM, 50 mM, 40 mM, 30 mM 20 mM, 10 mM, or less. The volume of the solution can be greater than about 1 ml, 2 ml, 3 ml, 5 ml, 10 ml, 20 ml, 30 ml, 50 ml, 100 ml, 200 ml, 300 ml, 500 ml, 1000 ml, 2000 ml, or more; less than 2000 ml, 1000 ml, 500 ml, 300 ml, 200 ml, 100 ml, 50 ml, 30 ml, 20 ml, 10 ml, 5 ml, 3 ml, 2 ml, 1 ml, or less; or within a range defined by any two of the preceding values.

### Transferring Polarization Using Radiofrequency Waveforms

Various embodiments of the present disclosure disclose applying a polarization transferring magnetic perturbation aimed to generate a magnetic field around the solution. In some embodiments, the magnetic field can have a strength of at least about 0.1 G, 0.2 G, 0.3 G, 0.4 G, 0.5 G, 0.6 G, 0.7 G, 0.8 G, 0.9 G, 1 G, 2 G, 3 G, 4 G, 5 G, 6 G, 7 G, 8 G, 9 G, 10 G, 20 G, 30 G, 40 G, 50 G, 60 G, 70 G, 80 G, 90 G, 100 G, 200 G, 300 G, 400 G, 500 G, 600 G, 700 G, 800 G, 900 G, 1,000 G, 2,000 G, 3,000 G, 4,000 G, 5,000 G, 6,000 G, 7,000 G, 8,000 G, 9,000 G, 10,000 G, 20,000 G, 30,000 G, 40,000 G, 50,000 G, 60,000 G, 70,000 G, 80,000 G, 90,000 G, 100,000 G, 200,000 G, or more; at most about 200,000 G, 100,000 G, 90,000 G, 80,000 G, 70,000 G, 60,000 G, 50,000 G, 40,000 G, 30,000 G, 20,000 G, 10,000 G, 9,000 G, 8,000 G, 7,000 G, 6,000 G, 5,000 G, 4,000 G, 3,000 G, 2,000 G, 1,000 G, 900 G, 800 G, 700 G, 600 G, 500 G. 400 G, 300 G. 200 G, 100 G, 90 G, 80 G, 70 G, 60 G, 50 G, 40 G, 30 G, 20 G, 10 G, 9 G, 8 G, 7 G, 6 G, 5 G, 4 G, 3 G, 2 G, 1 G, 0.9 G, 0.8 G, 0.7 G, 0.6 G, 0.5 G, 0.4 G, 0.3 G, 0.2 G, 0.1 G, or less (1G=10⁻⁴ T); or within a range defined by any two of the preceding values. For example, in some embodiments, the magnetic field has a strength of 0.1 G - 200,000 G around the solution. The magnetic perturbation can be produced by an electro-magnet or a permanent magnet. The magnetic field can be applied to the sample in pulses or in a continuous wave (CW). The magnetic perturbation can be static or time varying.

A signal generator can be configured to generate one or more radiofrequency (RF) waveforms that can be applied to the sample to transfer polarization. The signal generator can include one more computing unit, processors, controllers, associate memories, PCs, computers services, or any devices capable of carrying computational operations using inputs and producing outputs. In some embodiments, RF coils may radiate, or 'apply' the pulse sequences, including the first RF waveform. In some embodiments, the RF coils may have one or more channels. Channels may be pathways for RF signals. There may be provided at least one channel for each different type of NMR spectroscopy. In some embodiments, there may be at least one channel for ¹H and at least one channel for ¹³C. A first RF waveform can be applied to a ¹H channel of the one or more radiofrequency coils (RF coils) disposed around the sample. In some embodiments, a second RF waveform can be applied to a ¹³C channel of the RF coils. In some embodiments, the RF waveforms on the ¹H channel and ¹³C channel can be configured to apply a polarization transfer sequence, such as PH-INEPT, Goldman's sequence, S2M, S2hM, SLIC, ADAPT or ESOTERIC.

In some embodiments, the RF waveforms can be configured to support polarization transfer, even in the presence of a large proton full width half maximum (FWHM). Such RF waveforms can include a pulse sequence, which can include tens to hundreds of RF pulses. The sequence can be configured such that the pulses protect against the detrimental effects of magnetic field inhomogeneities on polarization transfer.

In some embodiments, a pulse sequence for polarization may be configured to transfer the spin order from non-equivalent two ¹H hydrogenated spins, e.g., when the chemical shift difference is larger than the J coupling between them. This is the case in some embodiments in the targeted magnetic fields of 100 mT-6000 mT, for example for many esters where the hydrogenation occurs on the sidearms. ESOTHERIC, for example, may be a pulse sequence suited for polarization transfer in this regime.

In some embodiments, the pulse sequence may be configured to transfer the spin order from equivalent ¹H hydrogen spins, e.g., when the chemical shift difference is smaller than the J coupling between them. Such pulse sequences may be used in magnetic fields greater than about 0.01 mT, 0.05 mT 0.1 mT, 0.2 mT, 0.3 mT, 0.4 mT, 0.5 mT, 0.6 mT, 0.7 mT, 0.8 mT, 0.9 mT, 1.0 mT, 2.0 mT, 3.0 mT, 4.0 mT, 5.0 mT, 6.0 mT, 7.0 mT, 8.0 mT, 9.0 mT, 10 mT, 20 mT, 30 mT, 40 mT, 50 mT, 60 mT, 70 mT, 80 mT, 90 mT, 100 mT, 200 mT, 300 mT, 400 mT, 500 mT, 600 mT, 700 mT, 800 mT, 900 mT, 1000 mT, 2000 mT, 3000 mT, 4000 mT, 5000 mT, 6000 mT, or more; less than about 6000 mT, 5000 mT, 4000 mT, 3000 mT, 2000 mT, 1000 mT, 900 mT, 800 mT, 700 mT, 600 mT, 500 mT, 300 mT, 200 mT, 100 mT, 90 mT, 80 mT, 70 mT, 60 mT, 50 mT, 40 mT, 30 mT, 20 mT, 10 mT, 9 mT, 8 mT, 7 mT, 6 mT, 5 mT, 4 mT, 3 mT, 2 mT, 1 mT, 0.9 mT, 0.8 mT, 0.7 mT, 0.6 mT, 0.5 mT, 0.4 mT, 0.3 mT, 0.2 mT, 0.1 mT, 0.05 mT, 0.01 mT or less; or within a range defined by any two of the preceding values. An example of such a sequence may be Goldman's sequence (M. Goldman, H. Jóhannesson, C. R. Phys. 2005, 6, 575-581. the singlet to heteronuclear magnetization (S2hM) sequence, or other sequences used in singlet NMR (e.g. ADAPT, SLIC, etc.). equivalent ¹H hydrogen spins, e.g., when the chemical shift difference is smaller than the J coupling between them may occur for hydrogenated protons in symmetric molecules independent of the magnetic field, or in many esters, in magnetic fields of less than about 100 mT, 90 mT, 80 mT, 70 mT, 60 mT, 50 mT, 40 mT, 30 mT, 20 mT, 10 mT, 9 mT, 8 mT, 7 mT, 6 mT, 5 mT, 4 mT, 3 mT, 2 mT, 1 mT, 0.9 mT, 0.8 mT, 0.7 mT, 0.6 mT, 0.5 mT, 0.4 mT, 0.3 mT, 0.2 mT, 0.1 mT or less.

In some embodiments, a magnetic shield can be configured to maintain a magnetic field applied to the solution at greater than about 0 mG, 0.1 mG, 0.2 mG, 0.3 mG, 0.4 mG, 0.5 mG, 0.6 mG, 0.7 mG, 0.8 mG, 0.9 mG, 1.0 mG, 2.0 mG, 3.0 mG, 4.0 mG, 5.0 mG, 6.0 mG, 7.0 mG, 8.0 mG, 9.0 mG, 10 mG, 20 mG, 30 mG, 40 mG, 50 mG, 60 mG, 70 mG, 80 mG, 90 mG, 100 mG, or more; less than about 100 mG, 90 mG, 80 mG, 70 mG, 60 mG, 50 mG, 40 mG, 30 mG, 20 mG, 10 mG, 9 mG, 8 mG, 7 mG, 6 mG, 5 mG, 4 mG, 3 mG, 2 mG, 1 mG, 0.9 mG, 0.8 mG, 0.7 mG, 0.6 mG, 0.5 mG, 0.4 mG, 0.3 mG, 0.2 mG, 0.1 mG or less; or within a range defined by any two of the preceding values. The magnetic shield can maintain the magnetic field strength within the polarization chamber at such amplitudes during application of the polarization waveform to the one or more radiofrequency coils.

Consistent with disclosed embodiments, when performing PHIP or PHIP-SAH polarization, the RF waveform can be applied to a solution containing a parahydrogenated precursor or parahydrogenated target compound. When performing SABRE polarization, the RF waveform can be applied to a solution containing the target compound, parahydrogen, and a polarization transfer catalyst. The concentrations of the target compound, parahydrogen, and a polarization transfer catalyst can be as given herein.

### Transferring Polarization using Magnetic Field Modulation

In some embodiments, the polarization transfer magnetic perturbation can be performed in a magnetic shield (e.g. a mu shield, or the like) to achieve a homogenous, low magnetic field. The magnetic shield enables performance of polarization transfer to ¹³C nuclear spins at µT magnetic fields, below the earth's magnetic field. The low magnetic field can be greater than about 0 mG, 0.1 mG, 0.2 mG, 0.3 mG, 0.4 mG, 0.5 mG, 0.6 mG, 0.7 mG, 0.8 mG, 0.9 mG, 1.0 mG, 2.0 mG, 3.0 mG, 4.0 mG, 5.0 mG, 6.0 mG, 7.0 mG, 8.0 mG, 9.0 mG, 10 mG, 20 mG, 30 mG, 40 mG, 50 mG, 60 mG, 70 mG, 80 mG, 90 mG, 100 mG, or more; or less than about 100 mG, 90 mG, 80 mG, 70 mG, 60 mG, 50 mG, 40 mG, 30 mG, 20 mG, 10 mG, 9 mG, 8 mG, 7 mG, 6 mG, 5 mG, 4 mG, 3 mG, 2 mG, 1 mG, 0.9 mG, 0.8 mG, 0.7 mG, 0.6 mG, 0.5 mG, 0.4 mG, 0.3 mG, 0.2 mG, 0.1 mG or less; or within a range defined by any two of the preceding values. At these fields, the polarization is transferred by utilizing level avoided crossings (LAC) between the proton spins and other spin species of interest, including ¹³C, ¹⁵N, ¹⁹F, ³¹P. In some embodiments, the magnetic field can be tuned to a specific magnetic field strength for the LAC. In various embodiments, to enable robust polarization transfer in larger-volume samples, the magnetic field strength can be temporally modulated. For example, the magnetic field strength can be swept through the LAC conditions. Alternatively or additionally, the sample can be physically moved inside the magnetic field. Such modulation can relax constraints on magnetic field homogeneity and on magnetic field offsets. Thus robust polarization transfer can be performed at larger volumes and with greater efficiency. Furthermore, relaxing the constraints on magnetic field homogeneity and on magnetic field offsets can permit using of less complex, precise, or expensive polarization systems.

A lower bound of the magnetic field modulation can be greater than about -10 µT, -9 µT, -8 µT, -7 µT, -6 µT, -5 µT, -4 µT, -3 µT, -2 µT, -1 µT, -0.9 µT, -0.8 µT, -0.7 µT, -0.6 µT, -0.5 µT, -0.4 µT, -0.3 µT, -0.2 µT, -0.1 µT, or more; less than about -0.1 µT, -0.2 µT, -0.3 µT, -0.4 µT, -0.5 µT, -0.6 µT, -0.7 µT, -0.8 µT, -0.9 µT, -1 µT, -2 µT, -3 µT, -4 µT, -5 µT, -6 µT, - 7 µT, -8 µT, -9 µT, -10 µT, or less; or within a range defined by any two of the preceding values. A upper bound of the modulation can be greater than about 0.1 µT, 0.2 µT, 0.3 µT, 0.4 µT, 0.5 µT, 0.6 µT, 0.7 µT, 0.8 µT, 0.9 µT, 1 µT, 2 µT, 3 µT, 4 µT, 5 µT, 6 µT, 7 µT, 8 µT, 9 µT, 10 µT, or more; less than about 10 µT, 9 µT, 8 µT, 7 µT, 6 µT, 5 µT, 4 µT, 3 µT, 2 µT, 1 µT, 0.9 µT, 0.8 µT, 0.7 µT, 0.6 µT, 0.5 µT, 0.4 µT, 0.3 µT, 0.2 µT, 0.1 µT, or less; or within a range defined by any two of the preceding values. The magnetic field can have such an amplitude over a volume of, or including, the polarization transfer chamber 402. This volume can be greater than about 1 ml, 2 ml, 3 ml, 5 ml, 10 ml, 20 ml, 30 ml, 50 ml, 100 ml, 200 ml, 300 ml, 500 ml, 1000 ml, 2000 ml, or more; less than 2000 ml, 1000 ml, 500 ml, 300 ml, 200 ml, 100 ml, 50 ml, 30 ml, 20 ml, 10 ml, 5 ml, 3 ml, 2 ml, 1 ml, or less; or within a range defined by any two of the preceding values. The modulation is can be performed over a duration. The duration can be greater than about 100 ms, 200 ms, 300 ms, 500 ms, 1000 ms, 2000 ms, 3000 ms, 5000 ms, 10000 ms, 20000 ms, 30000 ms, or more; less than about 30000 ms, 20000 ms, 10000 ms, 5000 ms, 3000 ms, 2000 ms, 1000 ms, 500 ms, 300 ms, 200 ms, 100 ms, or less; or within a range defined by any two of the preceding values. Accordingly, the rate of change of the amplitude of the magnetic field can be greater than about 0.05 µT, 0.1 µT, 0.5 µT, 1 µT, or more. The upper bound on the rate of change of the amplitude of the magnetic field may be determined by the capabilities of the equipment used to perform the sweep. In some embodiments, when the magnetic field is within the upper and lower bounds, disclosed above, the spatial deviation of the magnetic field over the volume during modulation can be less than about half (or a quarter, or an eighth, or a tenth) of the amplitude of the magnetic field. For example, when the magnetic field strength is less than 2 µT (or greater than - 2 µT) then the spatial deviation of the magnetic field over the volume during modulation can be less than 1 uT (or less than 0.5 µT, 0.25 µT, or 0.2 µT). As an additional example, when the magnetic field strength is less than 10 µT (or greater than - 10 µT) then the spatial deviation of the magnetic field over the volume during modulation can be less than 5 uT (or less than 2.5 µT, 1.25 µT, or 1.0 µT). The spatial deviation can be measured for example by taking at least 10, 30, 50, 100, 500 spatially randomly sampled or spatially equally distributed measurements of the magnetic field within the volume and calculating the standard deviation of the sampled magnetic field measurements. Such homogeneity can be achieved for example in a large homogeneous magnetic shield by having a large piercing solenoid through the magnetic shield or by using large Helmholtz coils with a large homogeneous region for producing the magnetic field amplitude modulation. In certain embodiments the modulation is a sweep of the magnetic field. In some embodiments, the magnetic field amplitude modulation includes a diabatic jump, monotonous amplitude variation or combinations thereof.

In certain embodiments, following the polarization transfer step, the non-hydrogen nuclear spin of the biorelevant imaging agent has nuclear spin polarization more than 1%, 5%, 10%, 15%, 20%, 25%, 30%, 35%, 40%, 45%, 50%, or more; less than 50%, 45%, 40%, 35%, 30%, 25%, 20%, 15%, 10%, 5%, 1% or less; or within a range defined by any two of the preceding values. In some embodiments this polarization is achieved for a solution volume larger than about 1 ml, 2 ml, 3 ml, 4 ml, 5 ml, 6 ml, 7 ml, 8 ml, 9 ml, 10 ml, 20 ml, 30 ml, 40 ml, 50 ml, 60 ml, 70 ml, 80 ml, 90 ml, 100 ml, 200 ml, 300 ml, 400 ml, 500 ml, or more; or less than about 500 ml, 400 ml, 300 ml, 200 ml, 100 ml, 90 ml, 80 ml, 70 ml, 60 ml, 50 ml, 40 ml, 30 ml, 20 ml, 10 ml, 9 ml, 8 ml, 7 ml, 6 ml, 5 ml, 4 ml, 3 ml, 2 ml, 1 ml, or less; or within a range defined by any two of the preceding values. In various embodiments, following polarization transfer a portion of the population difference in parahydrogenated proton spin states has been transferred to polarization of the target (e.g., ¹³C) nuclear spin of the biorelevant imaging agent. This portion can be more than 1%, 5%, 10%, 15%, 20%, 25%, 30%, 35%, 40%, 45%, 50%, or more; less than 50%, 45%, 40%, 35%, 30%, 25%, 20%, 15%, 10%, 5%, 1% or less; or within a range defined by any two of the preceding values.

In certain embodiments, the magnetic field modulation includes a diabatic jump of the magnetic field, where a jump is defined as a change of the magnetic field faster than 1 µT, 5 µT, 10 µT per second. The diabatic jump can be performed to a magnetic field where a level avoided crossing including the proton spins and a non-proton spin occur. Given the J couplings between the nuclear spins in the system, this value can be calculated analytically or identified by plotting the energy levels of the Hamiltonian for different magnetic fields, and identifying the LAC. In some embodiments, the duration where the magnetic field amplitude is at the LAC condition is less than 2 seconds, 1 second, 0.5 seconds.

In certain embodiments, modulation of the amplitude of the magnetic field can include varying the magnetic field amplitude monotonically (or monotonically over each of a limited number of interval - such as one to ten increasing interval and/or one to ten decreasing intervals). In various embodiments, the modulation of the amplitude of the magnetic field comprises linearly varying the amplitude of the magnetic field. The initial magnetic field amplitude of the sweep, the end magnetic field amplitude and the total duration of the sweep can be optimized for the target molecule. In some embodiments the magnetic field amplitude during the sweep is within a lower bound and an upper bound. The lower bound can be greater than about -2 µT, -1 µT, -0.9 µT, -0.8 µT, -0.7 µT, -0.6 µT, -0.5 µT, -0.4 µT, -0.3 µT, -0.2 µT, -0.1 µT, or more; less than about -0.1 µT, -0.2 µT, -0.3 µT, -0.4 µT, -0.5 µT, -0.6 µT, -0.7 µT, -0.8 µT, -0.9 ¡.tT, -1 µT, -2 µT, or less; or within a range defined by any two of the preceding values. The upper bound can be greater than about 0.1 µT, 0.2 µT, 0.3 µT, 0.4 µT, 0.5 µT, 0.6 µT, 0.7 µT, 0.8 µT, 0.9 µT, 1 µT, 2 µT, or more; less than about 2 µT, 1 µT, 0.9 µT, 0.8 µT, 0.7 µT, 0.6 µT, 0.5 µT, 0.4 µT, 0.3 µT, 0.2 µT, 0.1 µT, or less; or within a range defined by any two of the preceding values. In some embodiments the duration of modulation can be greater than about 100 ms, 200 ms, 300 ms, 500 ms, 1000 ms, 2000 ms, 3000 ms, 5000 ms, 10000 ms, or more; less than about 10000 ms, 5000 ms, 3000 ms, 2000 ms, 1000 ms, 500 ms, 300 ms, 200 ms, 100 ms, or less; or within a range defined by any two of the preceding values. In some embodiments, the rate of amplitude change is varied along the amplitude profile. In some embodiments, a constant-adiabaticity sweep is calculated by choosing a certain subset of level avoided crossings of the spin system. In certain embodiments, the magnetic amplitude modulation includes a combination of diabatic jumps, monotonous amplitude modulation and rate of change sign reversals.

In some embodiments, certain magnetic field amplitudes can support spontaneous spin order transfer between parahydrogen and the target compound, when both are bound to a polarization catalyst. For example, SABRE-SHEATH can support such spin order transfer for 15N or 13C polarization using magnetic fields greater than about 100 nT, 200 nT, 300 nT, 400 nT, 500 nT, 600 nT, 700 nT, 800 nT, 900 nT, 1 mT, or more; less than about 1 mT, 900 nT, 800 nT, 700 nT, 600 nT, 500 nT, 400 nT, 300 nT, 200 nT, 100 nT, or less; or within a range defined by any two of the preceding values. As an additional example, SABRE-SHEATH can support such spin order transfer for ¹H polarization using magnetic fields above 0.1 mT and below 100 mT. The spin order of the parahydrogen may be transferred to polarization on the target molecule, or to spin order on the target molecule. In some embodiments using SABRE, the magnetic field value is changed between two or more fixed values to create an average dynamics leading to polarization transfer.

In alternative embodiments, the polarization transfer occurs at a magnetic field higher than 0.1 G and below 200,000 G by applying a transferring waveform utilizing RF continuous wave or pulses to transfer the spin order from the bound parahydrogen to polarization on a nuclear spin in the catalyst-bound target molecule. The SLIC-SABRE, SABRE-INEPT or ADAPT sequences can be used for example in high-field SABRE transfer.

In another embodiment, the polarization of ¹H nuclei on a SABRE-polarized molecule, polarized either directly or by polarization transfer from another nuclei on the same molecule, is transferred to target molecules by chemical exchange, for example using the SABRE-RELAY method.

### Purification and Separation

In some embodiments, the precursor may be chosen such that following the hydrogenation and other potential chemical reactions, one of the products is a biorelevant imaging agent usable in HP MRI applications. In some embodiments, the biorelevant imaging agent can be produced through additional chemical reactions following hydrogenation. Such additional chemical reactions may include cleaving of a sidearm of the molecule, e.g., by hydrolysis. For example, the ester of the biorelevant imaging agent may be used for polarization using the PHIP-SAH method. Following hydrogenation and polarization transfer, the ester may be cleaved to produce the hyperpolarized biorelevant imaging agent.

The volume of solution including the target compound, following cleavage (and concentration of the target compound produced) can depend on the volume of the solution used for polarization transfer and concentration of the precursor in that solution. Exemplary ranges of solution volumes and precursor concentrations are described herein. As further specific examples, at least 1 ml of solution including at least 10 mM of the target compound can be produced. Alternatively, at least 5 ml of solution including at least 50 mM of the target compound can be produced. Alternatively, at least 10 ml of solution including at least 100 mM of the target compound can be produced.

Consistent with disclosed embodiments, following polarization transfer (and, in PHIP-SAH embodiments, cleaving of the precursor to generate the hyperpolarized target compound) the properties of the solution containing the hyperpolarized target compound can be modified to induce precipitation of the hyperpolarized target compound.

Such precipitation can enable separation of the hyperpolarized target compound (or the hyperpolarized precursor) from other substances in the solution (e.g., sidearm fragments in embodiments using PHIP-SAH, hydrogenation catalysts, hyperpolarization catalysts in embodiments using SABRE, or the like). The precipitate can form crystals, amorphous solid particles, polycrystal material, or the like. Following the precipitation, at least a fraction of the solid hyperpolarized target compounds (or the hyperpolarized precursor) can be separated from the solution and other substances in the solution. For example, a mixture of the precipitate and solution can be filtered to remove the particles. The filtered precipitate can be washed using a second solvent. The second solvent can be selected to remove residue of the original solvent and the other substances in the solution without fully dissolving the filtered precipitate. In some embodiments the washing step occurs in less than about 300 seconds, 200 seconds, 100 seconds, 90 seconds, 80 seconds, 70 seconds, 60 seconds, 50 seconds, 30 seconds, 20 seconds, 10 seconds; in more than about 10 seconds, 20 seconds, 30 seconds, 50 seconds, 60 seconds, 70 seconds, 80 seconds, 90 seconds, 100 seconds, 200 seconds, 300 seconds, or more; or within a range defined by any two of the preceding values. Precipitation can be performed using an aqueous solution (not covered by the scope of the invention) or, in accordance with the invention, a solution including an organic solvent, such as acetic acid, acetone, acetonitrile, benzene, 1-butanol, 2-butanol, 2-butanone, t-butyl alcohol, carbon tetrachloride, chlorobenzene, chloroform, cyclohexane, 1,2-dichloroethane, diethylene glycol, diethyl ether, diglyme (diethylene glycol dimethyl ether), 1,2-dimethoxyethane (glyme, DME), dimethylformamide (DMF), dimethyl sulfoxide (DMSO), 1,4-dioxane, ethanol, ethyl acetate, ethylene glycol, glycerin, heptane, hexamethylphosphoramide (HMPA), hexamethylphosphoroustriamide (HMPT), hexane, methanol, methyl t-butyl ether (MTBE), methylene chloride, N-methyl-2-pyrrolidinone (NMP), nitromethane, pentane, petroleum ether (ligroine), 1-propanol, 2-propanol, pyridine, tetrahydrofuran (THF), toluene, triethyl amine, water, heavy water, o-xylene, m-xylene, p-xylene. According to the invention, , an organic solvent is used for polarization and precipitation steps. For example, parahydrogen may be more soluble in a range of organic solvents than in an aqueous solution. Thus, hydrogenation can occur more efficiently in such solvents. Accordingly, in some embodiments using PHIP, parahydrogen-induced polarization can occurs in a solution formed with an organic solvent. In various embodiments not covered by the scope of the invention an aqueous solution is used for hydrogenation and the polarization transfer. In some embodiments, this aqueous solution can be mixed with a miscible organic solvent before precipitation of the hyperpolarized target compound.

In some embodiments, precipitation of the target compound can be induced by changing the pH of the solvent. In organic solvents, certain biorelevant imaging agents (e.g., carboxylic acids, pyridine, and the like) can be soluble in concentrations suitable for polarization using PHIP or SABRE. However, salts of these biorelevant imaging agents can be very insoluble. For example, such salts may be insoluble at concentrations below about 10 mM, 1 mM, 0.1 mM, 0.01 mM, or less; above about 0.01 mM. 0.1 mM, 1 mM, 10 mM, or more; or within a range defined by any two of the preceding values. An organic solution can contain a biorelevant imaging agent in a concentration suitable for hyperpolarization using PHIP or SABRE. Following polarization, the pH of the organic solution can be changed to induce precipitation of the salt of the biorelevant imaging agent. In aqueous solutions, the salt of certain biorelevant imaging agents (e.g., fumarate, glutamate, and the like) is more soluble than the acidic form. Thus an aqueous solution can contain a biorelevant imaging agent in a concentration suitable for hyperpolarization using PHIP or SABRE. Following polarization, the pH of the aqueous solution can be lowered to induce precipitation of the acidic form of the biorelevant imaging agent.

Consistent with disclosed embodiments, the change of pH can be induced by addition of an acidic or a basic molecule to the solution, such as sodium chloride or sodium hydroxide. In some embodiments, the change of pH can be induced by mixing the solution with another solution that has a substantially different pH. In some embodiments the precipitation due to change of pH occurs in less than about 100 seconds, 90 seconds, 80 seconds, 70 seconds, 60 seconds, 50 seconds, 40 seconds, 30 seconds, 20 seconds, 10 seconds, 9 seconds, 8 seconds, 7 seconds, 6 seconds, 5 seconds, 4 seconds, 3 seconds, 2 second, 1 second, or less; or more than about 1 second, 2 seconds, 3 seconds, 4 seconds, 5 seconds, 6 seconds, 7 seconds, 8 seconds, 9 seconds, 10 second, 20 seconds, 30 seconds, 40 seconds, 50 seconds, 60 seconds, 70 seconds, 80 seconds, 90 seconds, 100 seconds, or more; or within a range defined by any two of the preceding values.

In some embodiments, precipitation of the target compound can be induced without changing the pH of the solution of inducing the hyperpolarized target compound to change between a salt and an acid form.

In some embodiments, hydrogenation and polarization transfer can occur in a first solution having a first solvent. The target compound may have a high solubility in the first solvent. Precipitation can be induced by mixing the first solution with a second solvent to form a second solution. The second solvent can be chosen such that the solubility of the target compound in the second solution is low enough to initiate precipitation.

In some embodiments, the temperature of the solution is changed, thereby lowering the solubility of the hyperpolarized molecules and initiating precipitation. In most solvents the solubility decreases as the temperature decreases. The change in temperature to the desired temperature with the lower solubility is preferably performed within less than about 100 seconds, 50 seconds, 30 seconds, 20 seconds, 10 seconds, 5 seconds, 3 seconds, 2 second, 1 second, or less; within more than about 1 second, 2 seconds, 3 seconds, 5 seconds, 10 second, 20 seconds, 30 seconds, 50 seconds, 100 seconds, or more; or within a range defined by any two of the preceding values. For example, the maximal mole fraction of acetic acid in n-heptane is 0.935 at 14.8 °C, only 0.02 at -29.2 °C and lower at even colder temperatures. The selected temperature for precipitation can be selected as a temperature above the freezing point of the solvent.

In some embodiments, the surface area of the solution can be increased to induce nucleation of the precipitate. This can be achieved for example by spraying the solution through a nozzle to create very small droplets with a high surface area. In certain embodiments, microcrystal seeds (which may be of the target compound, or another biocompatible compound) can be added to the solution to induce precipitation.

In some embodiments, the pressure of the solution is changed, thereby lowering the solubility of the hyperpolarized compounds and initiating precipitation.

In some embodiments, the concentration of the hyperpolarized target compound is raised above its solubility limit, thereby inducing precipitation without lowering the compound solubility level in the solvent. This can be achieved for example by adding unpolarized molecules of the target compound or by evaporating a certain volume of the solvent, thereby increasing the concentration of the target compound above its solubility limit.

In some embodiments, the parameters for the PHIP or SABRE polarization can be optimized for a high concentration, preferably above the solubility limit for the target compound in its acid or salt form, even at the expense of achieving a lower polarization. This can be achieved for example in PHIP by starting with a high concentration of the precursors for hydrogenation and choosing a long hydrogenation time, which can cause polarization to be lowered due to relaxation, to achieve a high target compound concentration. In SABRE this can be achieved by increasing the concentration of the target compounds, at the expense that the polarization transfer from the parahydrogen to be less efficient.

In some embodiments the precipitation can be accelerated by the addition of mechanical energy or improved mixing of the mixture for a certain duration. This can be performed for example by applying ultrasonic waves on the mixture through an ultrasound solicitor, or by mechanical or magnetic mixing of the sample. In certain embodiments, the additional mixing or introduction of mechanical energy is performed for greater than about 0.1 seconds, 1 second, 5 seconds, 10 seconds, or more; less than about 10 seconds, 5 seconds, 1 second, 0.1 seconds, or less; or within a range defined by any two of the preceding values.

In some embodiments, precipitation can be induced by a chemical reaction involving a hyperpolarized target compound. The hyperpolarized target compound can react with another compound or in response to an external stimulus such as electromagnetic radiation (e.g., UV-irradiation). The product of this reaction may have a reduced solubility in comparison to the hyperpolarized target compound, thereby inducing the precipitation. For example, the external stimulus can modify a structure of the hyperpolarized target compound, thereby reducing the solubility of the hyperpolarized target compound. Following redissolution of the precipitate, additional reactions can be performed to produce a desired final product (e.g., a biorelevant imaging agent, NMR material, or the like).

In some embodiments a hydrolysis of the precursor can induce the precipitation. In some embodiments, for example using PHIP-SAH, a solvent can be selected such that the precursor is more soluble than the target compound. The concentration of the precursor in the solution can be selected such that, following cleavage of the sidearm and generation of the target compound, the target compound precipitates out of the solution. In some such embodiments, the cleavage can be initiated by changing the pH of the solution. For example, the cleavage can be initiated by adding a base (e.g., sodium hydroxide or another suitable base). In some embodiments, the solution can be formed with an organic solvent and the cleavage can be performed under basic conditions. Following the cleavage, the less-soluble target compound undergoes rapid precipitation while preserving its polarization. The same solution can be used for hydrogenation, polarization transfer, and precipitation, or another solvent can be mixed into the solution used for hydrogenation and polarization transfer.

In some embodiments using PHIP-SAH, the precipitation of the precursor can occur before cleavage. Such embodiments may be suitable when the precursor is more stable than the target compound. Precipitation of the precursor can be induced by modifying the pH of the solution such that the solubility of the precursor is reduced, or by mixing in a solution or compound which lowers the solubility of the precursor. In such embodiments, cleavage of the precursor can be performed after re-dissolution of the precursor in a solvent. The precursor can be filtered and washed as described herein to remove other substances present in the original solution, such as hydrogenation catalysts. The precursor can then be reacted (e.g., by cleaving a sidearm) to form the target compound. The target compound can be separated from other reaction products by liquid-liquid extraction or by an additional precipitation step, consistent with the precipitation methods described herein. In some embodiments using PHIP-SAH, precipitation can occur after generation of the target compound from the precursor. Such precipitation can be performed according to the methods described herein.

In some embodiments, the conversion of spin order to polarization occurs before the compounds are made to solidify and precipitate. In other embodiments, this conversion happens after re-dissolution of the crystals with the target compounds.

In some embodiments, several steps of precipitation, washing and redissolution are performed. This could be advantageous in further purifying the target compounds, increasing the relaxation time of the precipitate or further separating the polarization and cleavage steps. For example, the first precipitation can be used for washing out the catalyst, while the second precipitation is of a crystal form with a longer relaxation time, and which can be used for transport. In another embodiment, the first precipitation is of an ester of a target compound following hydrogenation and polarization transfer, and the second precipitation is performed after the cleavage.

Consistent with disclosed embodiments, steps of precipitation (and optionally washing) can separate the hyperpolarized target compound (or hyperpolarized precursor) from other substances in the original solution (e.g., catalysts, the original solvent(s), reaction products, or the like). For example, most of the hydrogenation catalyst present in the original solution can be retained in the original solution following precipitation of the target compound. In some embodiments, the precipitate (optionally following washing) can retain less than about 1%, 0.1%, 0.01%, 0.001%, or less of the hydrogenation catalyst; or retain more than about 0.001%, 0.01%, 0.1%, 1% or more of the hydrogenation catalyst. Similarly, in embodiments using PHIP-SAH and cleavage of the precursor molecule before precipitation, the precipitate can retain less than about 1%, 0.1%, 0.01%, 0.001%, or less of the cleavage byproducts (e.g., the sidearm or other residues of the cleavage); retain more than about 0.001%, 0.01%, 0.1%, 1% or more of the cleavage byproducts; or retain an amount of the cleavage byproducts within a range defined by any two of the preceding values.

In some embodiments, the amount of the hyperpolarized particles in the precipitate is accumulated over several iterations of hydrogenation, polarization transfer and precipitation of additional target compounds.

Hyperpolarized solid particles, especially in micrometer size, can be a valuable resource for several applications. Some applications include using the microparticles as NMR/MRI tracers, for example when using microparticles which do not dissolve in aqueous solutions. In some embodiments, the solid hyperpolarized compound obtained using precipitation of the PHIP or SABRE hyperpolarized solution is dissolved in a solvent and used as a hyperpolarized agent in hyperpolarized NMR or MRI.

According to the invention, following the precipitation and separation from the original solvent (and potentially transport of the hyperpolarized precipitate), the precipitate can be redissolved in a solvent (e.g., for use as an agent in hyperpolarized MRI). Preferably the solvent is a biocompatible solvent, preferably an aqueous solution. In some embodiments the redissolution is performed in less than about 60 seconds, 30 seconds, 20 seconds, 10 seconds, 5 seconds; more than about 5 seconds, 10 seconds, 20 seconds, 30 seconds, 60 seconds, or more; or within a range defined by any two of the preceding values.

In some embodiments, following re-dissolution, the target compound can be present in a higher concentration than the concentration used during the PHIP/SABRE polarization. In some embodiments the concentration of the hyperpolarized target compound after re-dissolution is greater than about 100 mM, 150 mM, 200 mM, 250 mM, 300 mM, 350 mM, 400 mM, 450 mM, 500 mM, or more, less than about 500 mM, 450 mM, 400 mM, 350 mM, 300 mM, 250 mM, 200 mM, 150 mM, 100 mM, or less; or within a range defined by any two of the preceding values. Thus, the concentration of the precursor or target compound during polarization can be independent of the concentration of the molecules in the injection solution. The concentration of the precursor or target compound during polarization can be selected for efficient polarization transfer. In some embodiments, this polarization concentration can be less than the concentration of the hyperpolarized target compound after re-dissolution. For example, the polarization concentration can be less than about 300 mM, 200 mM, 150 mM, 140 mM, 130 mM, 120 mM, 110 mM, 100 mM, 90 mM, 80 mM, 70 mM, 60 mM, 50 mM, 40 mM, 30 mM, 20 mM, 10 mM, or less; or greater than about 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 200 mM, 300 mM, or more; or within a range defined by any two of the preceding values. In some embodiments, samples of the precipitate may exhibit more than about 30%, 20%, 10%, 5%, 1% or less polarization; or less than about 1%, 5%, 10%, 20%, 30% or more polarization. Following re-dissolution of the particulate, the concentration of catalysts, the precursor, or cleavage byproducts may each be less than 1 µM, 900 nM, 800 nM, 700 nM, 600 nM, 500 nM, 400 nM, 300 nM, 200 nM, 100 nM, 90 nM, 80 nM, 70 nM, 60 nM, 50 nM, 40 nM, 30 nM, 20 nM, 10 nM, 9 nM, 8 nM, 7 nM, 6 nM, 5 nM, 4 nM, 3 nM, 2 nM, 1 nM, or less; greater than 1 nM, 2 nM, 3 nM, 4 nM, 5 nM, 6 nM, 7 nM, 8 nM, 9 nM, 10 nM, 20 nM, 30 nM, 40 nM, 50 nM, 60 nM, 70 nM, 80 nM, 90 nM, 100 nM, 200 nM, 300 nM, 400 nM, 500 nM, 600 nM, 700 nM, 800 nM, 900 nM, 1 µM, or more; or within a range defined by any two of the preceding values. In some embodiments, the purity of the hyperpolarized target compound following redissolution is greater than 90%, 92%, 95%, 98%, 99%, or more; less than 99%, 98%, 95%, 92%, 90% or less; or within a range defined by any two of the preceding values. In various embodiments, at least a fraction of the hyperpolarized compounds can be separated from the cleaved sidearms, or other reaction byproducts, if such exist.

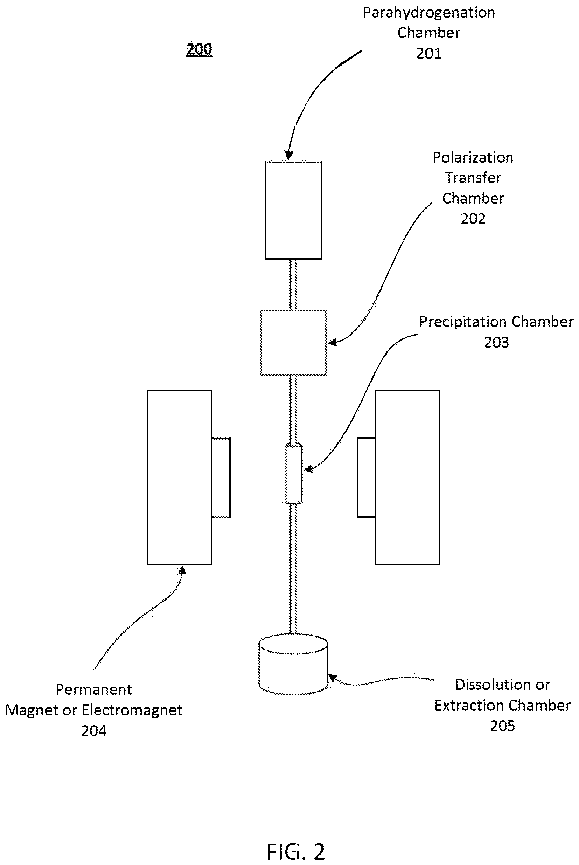

FIG. 2 depicts an exemplary schematic of a polarizer 200 for obtaining a high-concentration, biocompatible solution with hyperpolarized target compounds. Polarizer 200 can be used with PHIP, PHIP-SAH, or SABRE polarization methods. Parahydrogen can be dissolved in the solution containing the catalysts and target compounds in parahydrogenation chamber 201. Chamber 201 can be of a variety of designs known in the art to dissolve a large amount of parahydrogen in the solution, and achieve a high efficiency of hydrogenation or polarization transfer. In some embodiments, chamber 201 can include an inlet for introducing parahydrogen to the solution with a high pressure. In various embodiments, chamber 201 can be or include a flow chamber, in which a membrane separates the solution and flowing parahydrogen gas, and enables the parahydrogen to dissolve into the solution with a high surface area. In another embodiment chamber 201 can be a parahydrogen chamber, where the solution is sprayed into the chamber in small droplets, thereby establishing a large surface area for the dissolution of parahydrogen.

The transfer of spin order on the parahydrogen molecules to polarization on the target compounds can be performed in polarization transfer chamber 202. For example, for PHIP polarization, the polarization can be transferred by crossing a specific low magnetic field, typically lower than 1 µT, either during dissolving the hydrogen or afterwards. In another embodiment the magnetic field sweep includes at least some range between -10 µT and 10 µT. In another embodiment, a magnetic field of 0.1 G or above can be used and the polarization transfer is induced by a sequence of RF irradiation. In SABRE polarization, in a certain embodiment the polarization transfer occurs by dissolving the hydrogen in a specific magnetic field. This magnetic field can be lower than earth magnetic field, and therefore require a magnetic shield such as used in SABRE-SHEATH, or it can be higher than earth magnetic field such as used for 1H polarization via SABRE. In another embodiment, a magnetic field higher than 0.1 G is used and the polarization transfer is induced by a sequence of RF irradiation.

Precipitation chamber 203 can be used to induce precipitation of the hyperpolarized target compounds. In some embodiments, chamber 203 can include an inlet (e.g., a reagent port, or the like) for introducing a powder or solution (e.g., a precipitation agent) to mix with the solution containing the hyperpolarized target compound and induce the precipitation. In some embodiments, chamber 203 can include a stimulation port for introducing an electromagnetic radiation (e.g., ultraviolet radiation, or the like) into chamber 203. As described herein, the electromagnetic radiation can cause a solubility reducing change in the structure of the hyperpolarized target compound (e.g., thereby inducing precipitation of the hyperpolarized target compound). In some embodiments, the added powder or solution can include a base or acid, such as sodium hydroxide, potassium hydroxide, hydrogen chloride or sulfuric acid. In some embodiments the added powder or solution can induce a hydrolysis of the hyperpolarized compounds.

In some embodiments, chamber 203 can include a separation component for separating the precipitated compounds from a solution. In some embodiments, this component can be a filter which enables solvents to pass through but not microparticles. Example filters are commercially available sterile filters. In another embodiment, the separation component can include a centrifuge, which can be configured to centrifugally separate the precipitate from the solution (or separate different precipitates by particle size). In another embodiment, the precipitated crystals are given sufficient time to precipitate to the bottom of the separation component, preferably in a designed well, which enables the solution to be washed or diluted with little effect on the precipitated particles. In another embodiment, the separation component can include a heating element to heat evaporate the solution and preserve the precipitated particles. In another embodiment, the separation component can include temperature and pressure controllers to enable the sample to be separated by a freeze-drying method, including freezing the sample and sublimating the mixture excluding the precipitated particles.

In certain embodiments, the chamber 203 can include an inlet for introducing a solution for a washing step, wherein the solvent with the catalyst is washed away or significantly diluted with a washing fluid. In some embodiments the washing fluid is biocompatible. For example, if the separation component includes a filter, the washing fluid can flow through the filter, washing the precipitated particles from the original solvent.

A magnetic field generated by permanent magnet or electromagnet 204 can be applied to at least a portion of the precipitation chamber. In one embodiment, the magnetic field is applied to most or to all of the precipitation chamber. The magnetic field can be configured so that some of the precipitated particles are subject to a magnetic field of greater than about 1 mT, 10 mT, 100 mT, 1 T, 2 T, or more; or less than 2 T, 1 T, 100 mT, 10 mT, 1 mT, or less.

In some embodiments, a large volume of precipitated hyperpolarized particles can be accumulated by repeated iterations of parahydrogen dissolution, hyperpolarizing target molecules and precipitation. This can be performed in a continuous flow, in discrete batches or in a mix of continuous flow and discrete batches. In some embodiments, some of the steps are performed on several batches at once, while other steps are performed sequentially on each batch. This can be the case for example when one step can be performed on larger volumes than other steps. For example, in some embodiments, parahydrogen dissolution can be performed on a solution volume greater than about 5 ml, 20 ml, 50 ml, 100 ml or more (or less than about 100 ml, 50 ml, 20 ml, 5 ml, or less), while the polarization transfer can be performed on a smaller volume. In some embodiments the parahydrogen dissolution will be performed on a large volume of solution, while the polarization transfer will be performed sequentially on smaller batches. In this example the precipitation can be performed sequentially on each batch following polarization transfer or alternatively on larger batches following the accumulation of larger volumes of hyperpolarized target molecules following polarization transfer.

The particles are then dissolved in dissolution or extraction chamber 205, where they are re-dissolved and extracted for detection in hyperpolarized NMR or MRI. In some embodiments, the re-dissolution can be performed in a biocompatible solvent. In a preferred embodiment the solvent is an aqueous solution. In a preferred embodiment, the dissolution chamber includes an inlet for introducing the solvent. In a preferred embodiment the dissolution chamber includes an outlet. In another embodiment, the dissolved particles are injected directly from the dissolution chamber into the patient in a HP MRI scanner. In a preferred embodiment, a magnetic field generated by a permanent or electromagnet is applied to the dissolution chamber. The magnetic field in the region of the precipitated particles before dissolution can be greater than about 1 mT, 10 mT, 100 mT, 1 T, 2 T, or more; less than 2 T, 1 T, 100 mT, 10 mT, 1 mT, or less; or within a range defined by any two of the preceding values.

In some embodiments, chamber 203 and chamber 205 can be the same chamber. This chamber could be optionally transported between the precipitation and the dissolution to a different location. In some embodiments, chamber 201 and chamber 203 can be the same chamber.

### Transportation

Consistent with disclosed embodiments, polarization transfer and use of the target compound can occur at different locations. In various embodiments, the precursor (in the solution used for polarization transfer or as a precipitate) can be transported to another location following precipitation. In various embodiments, the target compound (in the solution used for polarization transfer, as a precipitate, or redissolved) can be transported to another location. The disclosed embodiments are not necessarily limited to any particular transport distance or duration. Instead, a maximum distance or duration can be determined based on the target molecule, the original degree or polarization, the required final degree of polarization, and the transport conditions. In some embodiments, the precipitate can be transported at least one meter in a suitable transportation device.

Consistent with disclosed embodiments, a transportation device can be configured to transport samples of the precursor or target compound. The transportation device can be arranged and configured for transporting one or more samples simultaneously. The transportation device can include a transport chamber configured to receive the one or more samples. The transportation device can be configured to maintain the transport chamber within a predetermined temperature range and a predetermined magnetic field strength. The transportation device can be configured to maintain the one or more samples in a magnetic field of more than about 10 G, 100 G, 1000 G, or more; or less than 1000 G, 100 G, 10 G, or less.

A permanent magnet or an electromagnet included in the transportation device can provide the magnetic field. Moreover, in some embodiments, the permanent magnet or electromagnet can be shielded to reduce the strength of the magnetic field outside the transportation device. The transportation device can also include a cooling system. The cooling system can be configured to maintain samples at a predetermined temperate or within a predetermined range of temperatures during transport. For example, the cooling system can be configured to maintain the samples at a temperature below 270 K, below 80 K, or below 4 K. In some embodiments, the transportation device can be configured to maintain the samples at approximately the temperature of liquid nitrogen. The transportation device can include insulation between the cooling system and the exterior of the transportation device, to minimize heat exchange with the external environment. In some embodiments, the cooling system can be configured to maintain the temperature of the samples using a cold gas flow. In various embodiments, the cooling system can be configured to maintain the temperature of the samples using a liquid coolant. In various embodiments, the transportation device can include a Dewar to provide cooling of the samples. In order to distribute the hyperpolarized samples also across large distances, the container preferably can be transported by standard transportation vehicles, such as planes, trains, trucks, cars and ships.

In some embodiments the hyperpolarized precipitated particles are transported in the transportation device. In some embodiments the relaxation time of the hyperpolarized precipitated particles in the transportation device is longer than about 1 minute, 10 minutes, 30 minutes, 1 hour, 3 hours, 10 hours, or more; or shorter than about 10 hours, 3 hours, 1 hour, 30 minutes, 10 minutes, 1 minute, or less.

### Exemplary Systems

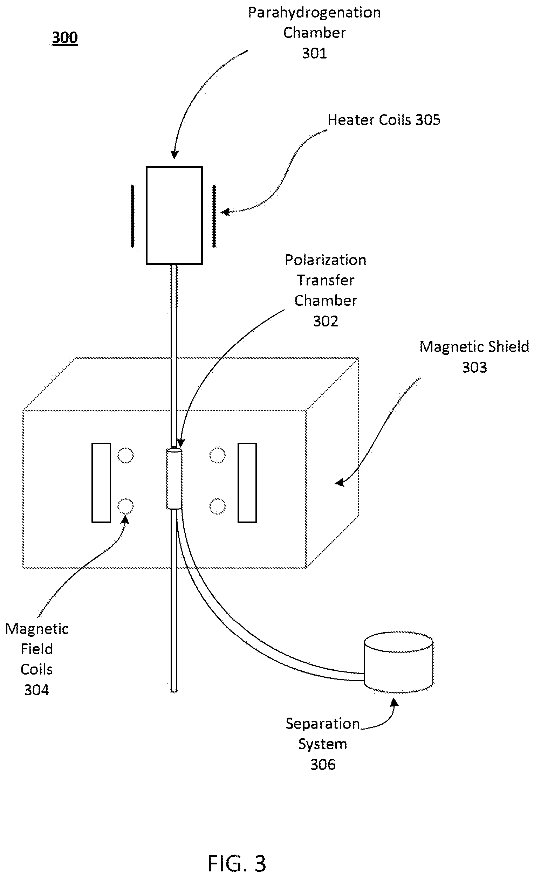

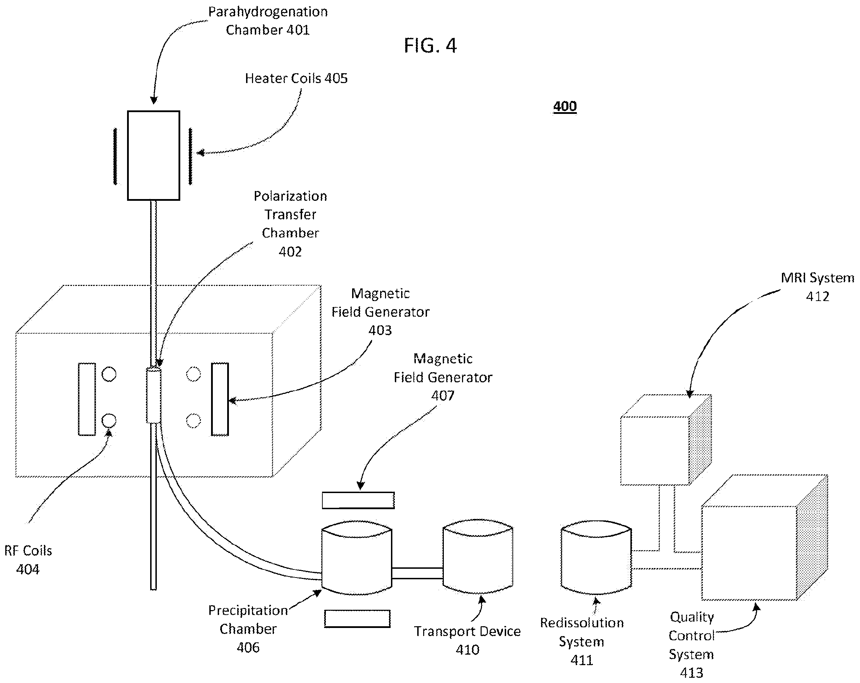

FIGs. 3 and 4 depict embodiments of large-scale polarizers, consistent with disclosed embodiments. These polarizers can be used for PHIP, PHIP-SAH, or SABRE polarization methods. FIG. 3 depicts an exemplary polarization system 300, consistent with disclosed embodiments. System 300 includes a parahydrogenation chamber 301, a polarization transfer chamber 302 inside magnetic shield 303, and magnetic field coils 304 capable of setting the magnetic field amplitude in the polarization transfer chamber 302 to a specific value.

Parahydrogen can be dissolved in a solution in parahydrogenation chamber 301. Chamber 301 can be designed and configured to accommodate a combination of the solution with a large amount of parahydrogen in the solution. Chamber 301 can be designed and configured to achieve a high efficiency of hydrogenation. In some embodiments, heating coils 305 can be disposed around or within chamber 301. Such coils can be energized to control the temperature of the solution within chamber 301.

The solution can contain a target compound and a polarization catalyst in SABRE embodiments, or a precursor to the target compound in PHIP or PHIP-SAH embodiments. The parahydrogen can be combined with the solution for (or the dissolution of the parahydrogen can occur in less than) a time interval. The time interval can be less than about 90 seconds, 60 seconds, 30 seconds, 20 seconds, 10 seconds, 5 seconds, or less; more than about 5 seconds, 10 seconds, 20 seconds, 30 seconds, 60 seconds, 90 seconds, or more; or within a range defined by any two of the preceding values. In certain embodiments, more than about 30 mM, 50 mM, 100 mM, 250 mM, 400 mM, 600 mM, 1000 mM, or more; less than about 1000 mM, 600 mM, 400 mM, 250 mM, 100 mM, 50 mM, 30 mM, or less; or within a range defined by any two of the preceding values of the precursor is hydrogenated.

In some embodiments, chamber 301 can include an inlet for introducing pressurized parahydrogen to the solution. In certain embodiments, the parahydrogen is introduced to solution in the hydrogenation chamber at a pressure at least about 10 bar, 15 bar, 20 bar, 25 bar, 30 bar, 35 bar, 40 bar, 45 bar, 50 bar, or more; at most about 50 bar, 45 bar, 40 bar, 35 bar, 30 bar, 25 bar, 20 bar, 15 bar, 10 bar, or less; or within a range defined by any two of the preceding values. In certain such embodiments the high-pressure dissolution of the parahydrogen into the solution occurs in a metallic chamber capable of withstanding the pressure at which the parahydrogen is introduced. In other embodiments the chamber is made out of a plastic material. In certain embodiments the bubbler includes a nozzle head which introduces the parahydrogen to the solution in microbubbles for larger surface areas.

In various embodiments, chamber 301 can be a flow chamber. In such embodiments, a membrane can separate the solution and flowing parahydrogen gas. The membrane can be arranged within the flow chamber to have a high surface area, to enable the parahydrogen to dissolve into the solution. In various embodiments, chamber 301 can include a chamber filled with parahydrogen gas. An aerosolizer in chamber 301 can be configured to spray the solution into the chamber in small droplets, thereby establishing a large surface area for the dissolution of parahydrogen.

As depicted in FIG. 3, system 300 can include polarization transfer chamber 302 inside magnetic shield 303 (constructed from mu-metal or another suitable shielding material). Magnetic shield 303 can enable application of a magnetic field of amplitude less than Earth magnetic field to a solution in the polarization transfer chamber 302. In certain embodiments, magnetic field coils 304 can enable setting the magnetic field in the polarization transfer chamber 302 to a specific value (or setting the magnetic field to follow a particular time-dependent amplitude trajectory). In certain embodiments, parahydrogenation chamber 301 can be outside magnetic shield 303, as shown in FIG. 3. Alternatively, parahydrogenation chamber 301 can be inside magnetic shield 303 (e.g., a single reservoir can combine functions of parahydrogenation chamber 301 and polarization transfer chamber 302). In some embodiments, the magnetic field coils 304 can be electrically connected and controlled by an external wave-form signal generator (not shown in FIG. 3). The external wave-form generator can be configured to generate a varying magnetic field amplitude in the magnetic shield to efficiently transfer the polarization. In some embodiments, magnetic field coils 304 can modulate an amplitude of the magnetic field within the polarization transfer chamber 302.

Consistent with disclosed embodiments, magnetic field coils 304 can modulate the magnetic field across a specified range between -10 µT and 10 µT in a volume between 1 ml and 2000 ml (e.g., as described herein). In certain embodiments, when the amplitude of the magnetic field during modulation is less than 10 uT the spatial deviation of the magnetic field during modulation is less that half (or a quarter, or an eighth, or a tenth) of the amplitude of the magnetic field (e.g., as described herein). Such homogeneity can be achieved for example in a large homogeneous magnetic shield by having a large piercing solenoid through the magnetic shield or by using large Helmholtz coils with a large homogeneous region for producing the magnetic field amplitude modulation.

The modulation can be performed over a duration. The duration can be between about 100 ms and 30000 ms (e.g., as described herein). In certain embodiments the modulation can be a sweep of the magnetic field. In some embodiments, the magnetic field coils are controlled by an external wave-form generator. The external wave-form generator is configured to generate a varying magnetic field in the magnetic shield to efficiently transfer the polarization. In certain embodiments, for example in SABRE polarization, the magnetic field in the polarization chamber 302 can be kept at a constant value or range less than about 1 µT, 2 µT, 3 µT, 4 µT, 5 µT, 6 µT, 7 µT, 8 µT, 9 µT, 10 µT, 20 µT, 30 µT, 40 µT, 50 µT, 60 µT, 70 µT, 80 µT, 90 µT, 100 µT, 200 µT, 300 µT, 400 µT, 500 µT, 600 µT, 700 µT, 800 µT, 900 µT, 1000 µT, or greater; a constant value or range greater than about 1000 µT, 900 µT, 800 µT, 700 µT, 600 µT, 500 µT, 400 µT, 300 µT, 200 µT, 100 µT, 90 µT, 80 µT, 70 µT, 60 µT, 50 µT, 40 µT, 30 µT, 20 µT, 10 µT, 9 µT, 8 µT, 7 µT, 6 µT, 5 µT, 4 µT, 3 µT, 2 µT, 1 µT, or less; or within a range defined by any two of the preceding values. The magnetic field in the polarization chamber 302 can be kept at the constant value or range for at least about 1 second, 2 seconds, 3 seconds, 4 seconds, 5 seconds, 6 seconds, 7 seconds, 8 seconds, 9 seconds, 10 seconds, 20 second, 30 seconds, 40 seconds, 50 seconds, 60 seconds or more; at most about 60 seconds, 50 seconds, 40 seconds, 30 seconds, 20 seconds, 10 seconds, 9 seconds, 8 seconds, 7 seconds, 6 seconds, 5 seconds, 4 seconds, 3 seconds, 2 seconds, 1 second, or less; or within a range defined by any two of the preceding values.

In certain embodiments, the magnetic field modulation can be performed by flowing the solution through an area inside the magnetic shield 303 with a constant magnetic field profile.

In certain embodiment a single solenoid can be used for controlling the magnetic field gradient in the entrance and exit to the magnetic shield 303, as well for modulating the magnetic field inside the shield.

Following polarization, the solution is conveyed to a separation system 306. In a preferred embodiment, the separation system 306 is a precipitation chamber, as detailed in section "Precipitation". In a preferred embodiment, the redissolution chamber, detailed in section "Precipitation", is a chamber in separation system 306. Accordingly, following extraction and purification of the hyperpolarized target compound, the hyperpolarized target compound can be dissolved in a biocompatible solvent, preferably an aqueous solvent. In another embodiment, a liquid-liquid extraction between an organic phase and an aqueous phase can be performed by separation system 306. Such a liquid-liquid extraction can be used to separate the target compound from catalysts, cleavage byproducts, or organic solvent(s) used for the hydrogenation or polarization transfer. In some embodiments, a fluorinated phase may be used in addition to/in alternative of the organic phase, and may be combined with an aqueous phase for improved separation. However, the invention is limited to the use of an organic solvent. In some other embodiments, purification step may include catalyst scavenging. Such catalyst scavenging can include adding a binding material which binds preferentially to the catalyst, with the binding material subsequently being separated by filtration, centrifugation, or other mechanical means. In some embodiments, the hyperpolarized molecules may be cleaved to produce the target molecules inside separation system 306. In some embodiments, pH level and temperature of the sample may be monitored and controlled in separation system 306 in order to meet conditions for injection into the patient. Alternatively, a quality control system (similar to quality control system 413) can be used monitor the characteristics of the sample. The sample can be injected to an animal or a human patient for hyperpolarized MRI scan in an MRI scanner (e.g., such as in MRI system 412).

FIG. 4 depicts a polarizer 400, consistent with disclosed embodiments. Polarizer 400 can include parahydrogenation chamber 401, polarization chamber 402, magnetic field generator 403, RF coils 404, heater coils 405, precipitation chamber 406, magnetic field generator 407, and transport device 410. Polarizer 400 can optionally be used with redissolution system 411, MRI system 412, and quality control system 413. Parahydrogenation chamber 401, polarization chamber 402, and heater coils 405 can resemble corresponding components of system 300, described with reference to FIG. 3.

In various embodiments, the magnetic field generator 403 can include an electromagnet or permanent magnet capable of generating a magnetic field greater than 0.1 mT, 0.2 mT, 0.3 mT, 0.4 mT, 0.5 mT, 0.6 mT, 0.7 mT, 0.8 mT, 0.9 mT, 1.0 mT, 2.0 mT, 3.0 mT, 4.0 mT, 5.0 mT, 6.0 mT, 7.0 mT, 8.0 mT, 9.0 mT, 10 mT, 20 mT, 30 mT, 40 mT, 50 mT, 60 mT, 70 mT, 80 mT, 90 mT, 100 mT, 200 mT, 300 mT, 400 mT, 500 mT, 600 mT, 700 mT, 800 mT, 900 mT, 1000 mT, 2000 mT, 3000 mT, 4000 mT, 5000 mT, 6000 mT, or more; or less than about 6000 mT, 5000 mT, 4000 mT, 3000 mT, 2000 mT, 1000 mT, 900 mT, 800 mT, 700 mT, 600 mT, 500 mT, 300 mT, 200 mT, 100 mT, 90 mT, 80 mT, 70 mT, 60 mT, 50 mT, 40 mT, 30 mT, 20 mT, 10 mT, 9 mT, 8 mT, 7 mT, 6 mT, 5 mT, 4 mT, 3 mT, 2 mT, 1 mT, 0.9 mT, 0.8 mT, 0.7 mT, 0.6 mT, 0.5 mT, 0.4 mT, 0.3 mT, 0.2 mT, 0.1 mT or less. The magnetic field can be applied to the sample in polarization chamber 402.

In some embodiments, RF coils 404 can be configured to generate a radiofrequency waveform on the sample in the chamber for polarization transfer. In various embodiments, the waveform is configured to be a polarization transfer waveform relevant for transferring polarization on the sample. The polarization transfer waveform can be chosen according to the type of compound, the magnetic field, and whether the compound is polarized by PHIP or by SABRE methods, as described herein.

Following polarization, the solution is conveyed to precipitation chamber 406. Precipitation chamber 406 can resemble precipitation chamber 203, discussed with regards to **FIG. 2****.** Magnetic field generator 407, which can be a permanent magnet or electromagnet can apply a magnetic field of greater than about 1 mT, 10 mT, 100 mT, 1000 mT or more; or less than about 1000 mT, 100 mT, 10 mT, 1 mT, or less, to at least a portion of the precipitate in the precipitation chamber 406. Following precipitation, the precipitate can be transported to a different location in transport device 410, which can include a magnetic field generator and cooling system as described herein. The magnetic field generator include a permanent or electromagnet configured to apply about 1 mT, 10 mT, 100 mT, 1000 mT or more; or less than about 1000 mT, 100 mT, 10 mT, 1 mT, or less, to at least some of the precipitated particles in the transport device 410. The hyperpolarized particles can be transported to a redissolution system 411, as detailed herein, where the precipitate can be redissolved in a biocompatible solvent, preferably an aqueous solvent.

Following extraction and purification, the solution properties are monitored in quality control system 413. In a preferred embodiment, the quality control system monitors the pH and temperature of the solution. In some embodiments the quality control system 413 monitors as well whether trace amounts of the original solvent in which the parahydrogen was dissolved, for example by a UV/VIS spectrometer. The solution containing the hyperpolarized target compound can be injected to an animal or a human patient for hyperpolarized MRI scan in an MRI system 412.

### Exemplary Parahydrogenation and Precipitation of Pyruvate

### Synthesis of (3-phenylpropargyl)pyruvate-1-¹³C

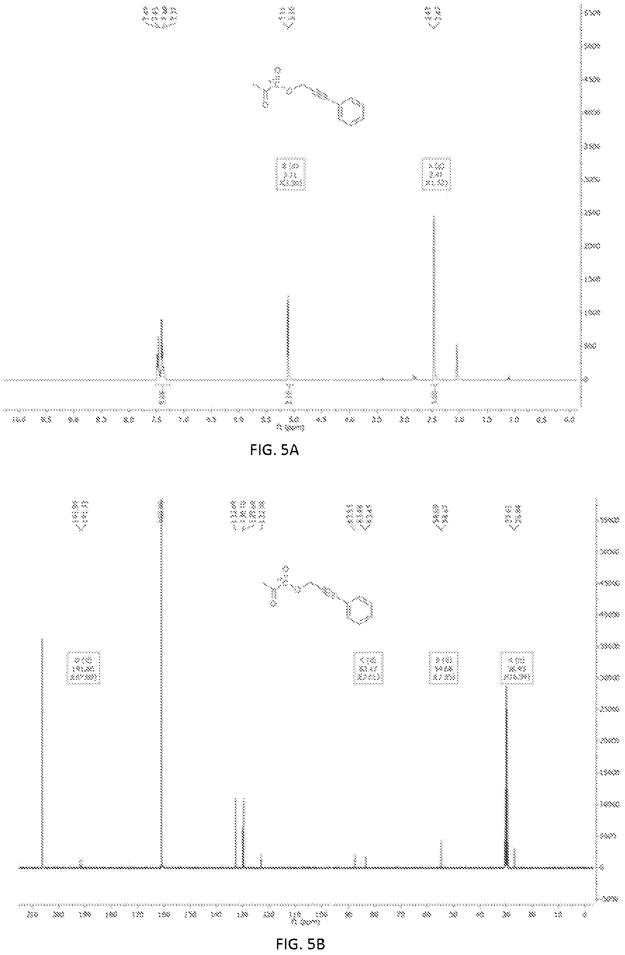

Pyruvic acid-1-¹³C (3.58 g, 40.2 mmol, Cambridge Isotope Laboratories) and 3-phenyl-2-propyn-1-ol (3.81 g, 28.8 mmol, Sigma Aldrich) were dissolved in anhydrous tetrahydrofuran (60 mL) and cooled to 0 °C with a water/ice bath. Anhydrous pyridine (5.70 g, 72.1 mmol, Sigma Aldrich) was added over a period of three minutes followed by the addition of methanesulfonyl chloride (4.62 g, 40.3 mmol, Sigma Aldrich) over a period of 15 minutes at 0 °C. After stirring for two hours at 0 °C and 3 hours at 20 °C, the reaction was quenched by pouring into 0 °C cold hydrochloric acid (0.5 M, 100 mL). The mixture was extracted with diethyl ether (3 x 100 mL), the combined organic extracts were washed with saturated aqueous sodium bicarbonate (100 mL) followed by washing with brine (100 mL). The organic phase was dried over sodium sulfate, the volatile components were removed under reduced pressure and the residue was filter over flash silica gel (cyclohexane/ethyl acetate = 9:1). The crude product was purified with HPLC (cyclohexane/ethyl acetate) to give the (3-phenylpropargyl)pyruvate-1-¹³C as a colorless solid (4.08 g, 20.2 mmol, 70%). **FIGs. 5A** and **5B** depict the ¹H NMR spectrum (400 MHz, acetone-d6) and the ¹³C NMR spectrum (100 MHz, acetone-d6) of the (3-phenylpropargyl)pyruvate-1-¹³C. These figures demonstrate the purity of the synthesized pyruvate ester.

### Parahydrogenation of (3-phenylpropargyl)pyruvate-1-¹³C

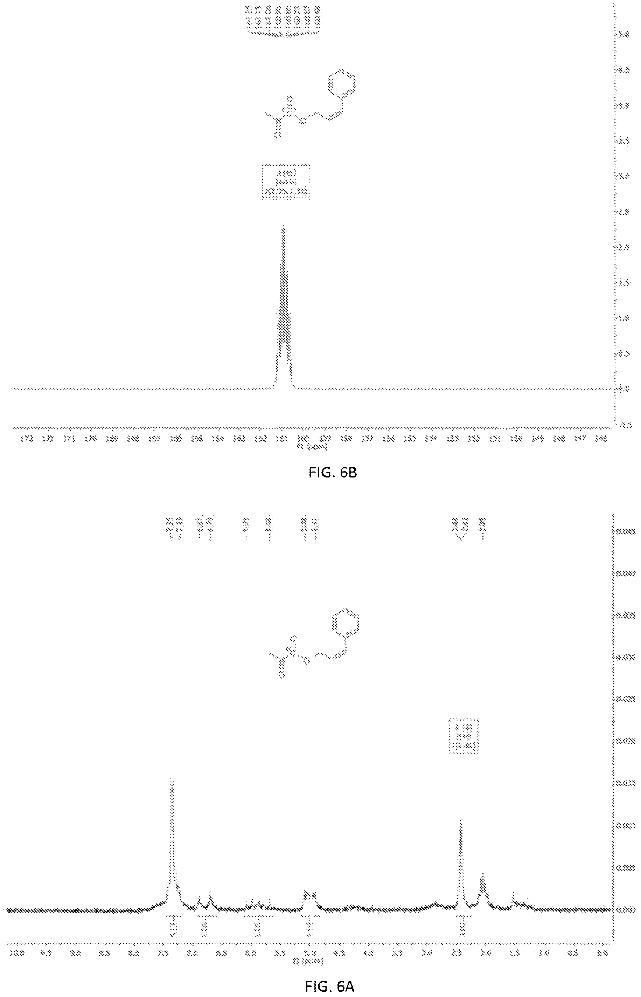

A solution of (3-phenylpropargyl)pyruvate-1-¹³C (100 mM in acetone-d6) was mixed with the hydrogenation catalyst [Rh(dppb)(COD)]BF₄ (5 mol%, Cas: 79255-71-3, Sigma Aldrich). The mixture was transferred in the reactor and parahydrogenation was carried out at 40 °C for 30 seconds with 20 bar parahydrogen. After hydrogenation, the mixture was transferred into the shield and a field sweep was performed for 4 seconds. Thereafter, the mixture was extracted from the shield and a ¹³C NMR spectrum was recorded immediately. **FIG. 6B** depicts the ¹³C NMR spectrum of hyperpolarized *E*-cinnamyl pyruvate-1-¹³C (20 MHz, acetone-d6), demonstrating the efficiency of the polarization transfer and **FIG. 6A** depicts the ¹H NMR spectrum of hydrogenated (3-phenylpropargyl)pyruvate-1-¹³C (80 MHz, acetone-d6), showing the successful hydrogenation of the precursor.

### Pyruvate ester cleavage and precipitation

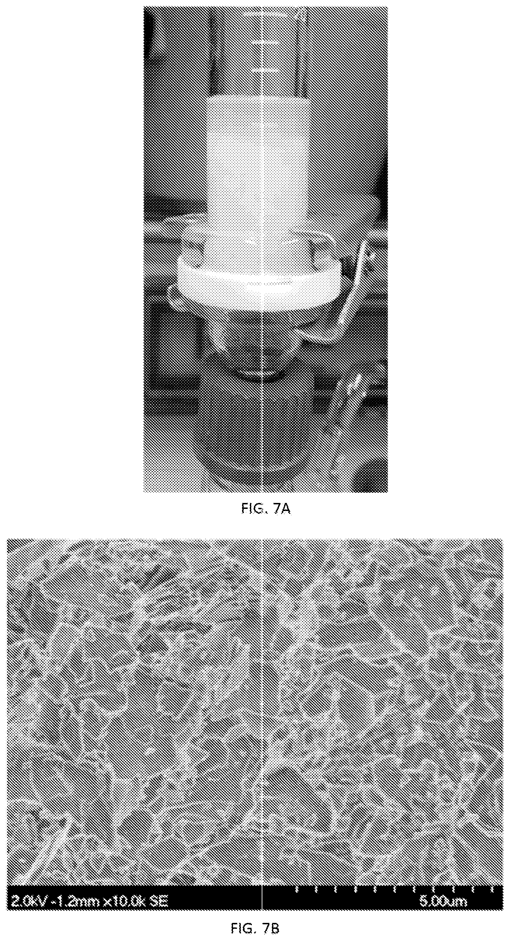

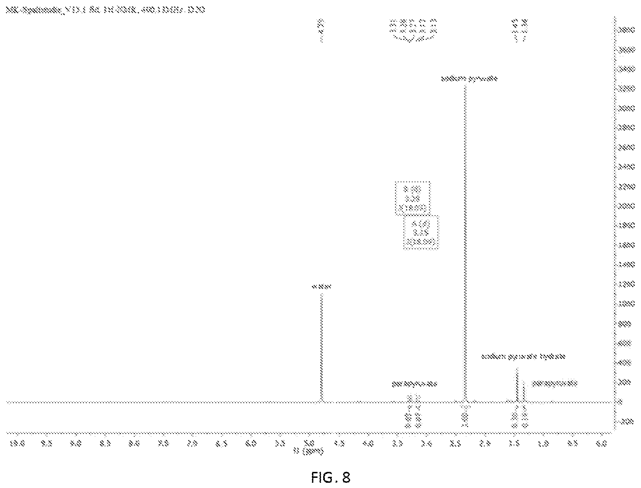

The solution of the pyruvate ester (e.g. ethyl pyruvate, cinnamyl pyruvate or (3-phenylpropargyl) pyruvate) in a 1:3 mixture of acetone/*n*-hexane (250 mM of pyruvate) is vigorously stirred at 20 °C. Over a period of 5-20 seconds, a solution of sodium hydroxide (200 mM in n-butanol, 0.7 equivalents) is added. A colorless precipitate forms during the first seconds of the addition of hydroxide. After complete addition of the sodium hydroxide solution, the mixture is poured onto a frit containing a filter, the liquid is sucked off under reduced pressure (12 mbar) and the remaining solid is washed with acetone and diethyl ether. **FIG. 7A** shows the mixture in the frit containing the filter. **FIG. 7B** shows a SEM image of precipitated and washed sodium pyruvate resulting from the cleavage of cinnamyl pyruvate with sodium hydroxide solution. **FIG. 8** depicts the ¹H NMR spectrum of precipitated and washed sodium pyruvate resulting from the cleavage of cinnamyl pyruvate with sodium hydroxide solution, after redissolution in D₂O (400.13 MHz, D₂O).

### Transport of Precipitate

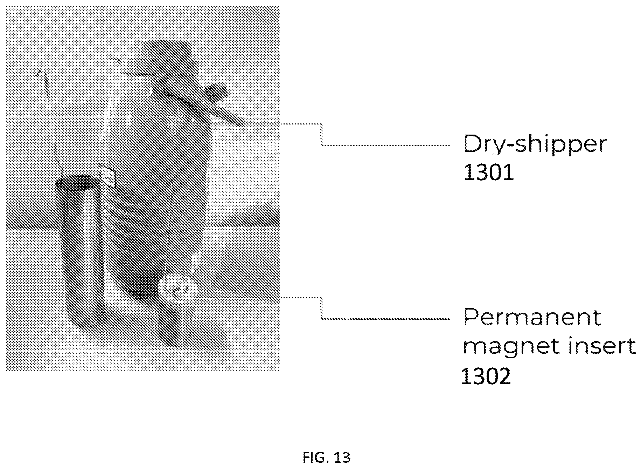

The precipitated sodium pyruvate particles can be transported in a holding magnet before redissolution. **FIG. 13** depicts a dry-shipper 1301 with a permanent magnet insert 1302. The permanent magnet provides a magnetic field of 0.75T within dry-shipper 1301. Dry-shipper 1301 can be suitable for transporting hyperpolarized precipitated particles before redissolution. Dry-shipper 1301 can be maintained at a suitable transportation temperature (e.g., temperature of approximately -190 C) for several days.

### Exemplary Parahydrogenation, Precipitation, and Use of Fumarate

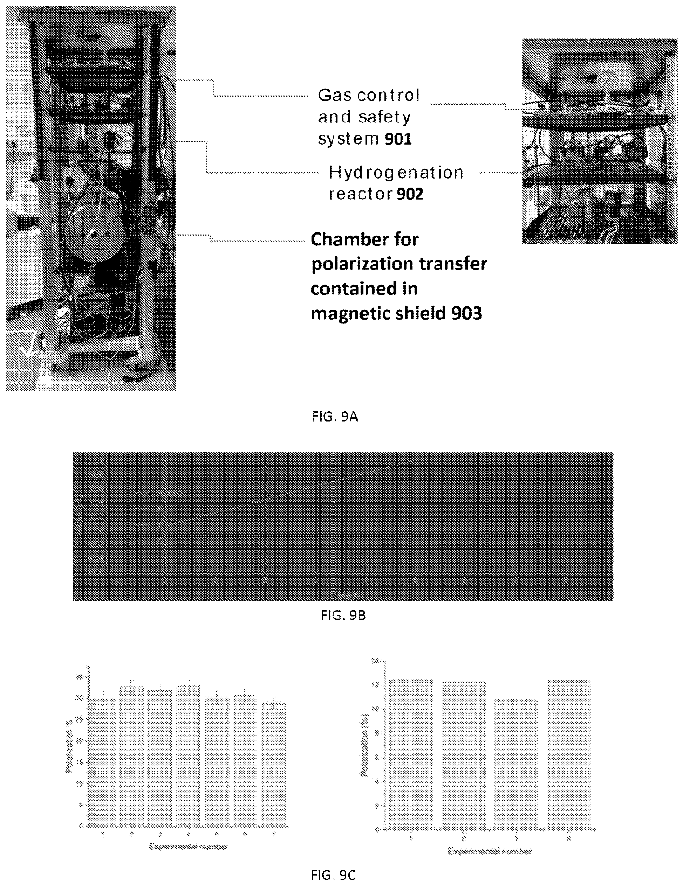

Experiments were performed on the experimental setup depicted in **FIG. 9A****.** The experiment setup includes a gas control and safety system 901, a hydrogenation reactor 902 (similar to parahydrogenation chamber 301), and a chamber for polarization transfer (similar to polarization transfer chamber 302) contained in a magnetic shield (similar to magnetic shield 303). The starting solution contains 400 mM acetylenedicarboxylic acid mono sodium salt, 250 mM sodium sulphite anhydrous and 8 mM tris(acetonitrile)pentamethylcyclopentadienyl ruthenium(ii) hexafluorophosphate catalyst. This solution is titrated with 1 M NaOD to a pH of 7.8. The solution is then filled in an ETFE coated aluminum reactor and heated to 90C. Subsequently the parahydrogen is bubbled though a frit into the solution at a pressure 10 bar and with a flow of 2 liter per minute. The hydrogenation is stopped after 20s and the solution is shuttled through a magnetic guide into the magnetic shield. Once the solution arrived and settled the vessel (approximately after 5 s) inside the magnetic shield a linear magnetic field sweep is carried out. The field sweep starts at 50 nT and ramps up to 1000 nT within 5 seconds. A magnetic shield (MS2, Twinleaf LLC, Princeton, U.S.) was used. The magnetic field shimming and sweeps were produced using a homemade solenoid piercing the shield top through bottom along the z-axis, powered by the analog output of a national instruments card (PCIe-6363,National Instruments US). **FIG. 9B** depicts the profile of the magnetic field sweep in the magnetic shield for transferring the population difference on the parahydrogenated spins to the ¹³C spin. The field sweep starts at 50 nT and ramps up to 1000 nT within 5 seconds.

The hydrogenated solution, containing 1-¹³C hyperpolarized sodium fumarate in excess of 100 mM, the hydrogenation catalyst, the unreacted starting material (acetylenedicarboxylic acid) and any by-products are transported into a fritted filter situated inside a 500mT magnetic field. The magnetic field must be large enough to prevent polarization loss in the solid phase (*i.e.* large compared to the local dipole fields produced by neighboring spins in the lattice, which typically requires a magnetic field >10mT).

The filter is prefilled with concentrated hydrochloric acid (¼ of the volume of the reaction mixture). Subsequently, the solution is exposed to ultrasound which instantly initiates the precipitation of fumaric acid. After two seconds, the liquid phase is sucked off (applying of 20 mbar vacuum, duration three seconds) which leaves the fumaric acid particles on the filter of the frit. Subsequently, two washing steps are applied: first, the solution is washed with 1 M hydrochloric acid in order to remove remaining catalyst impurities. Second, the solution is washed with acetone. All cleaning solvents are sucked off through the frit. After cleaning, the solution is redissolved in D₂O containing sodium bicarbonate (300 mM). The resulting solution contains 100-150 mM of sodium fumarate and has a physiological pH between 7 and 9. **FIG. 9C** shows initial results of the hyperpolarization achieved before and after precipitation and redissolution of the sodium fumerate. After further finetuning of the parameter processes, the hyperpolarization before precipitation was 45% and after precipitation and redissolution it was 25%.

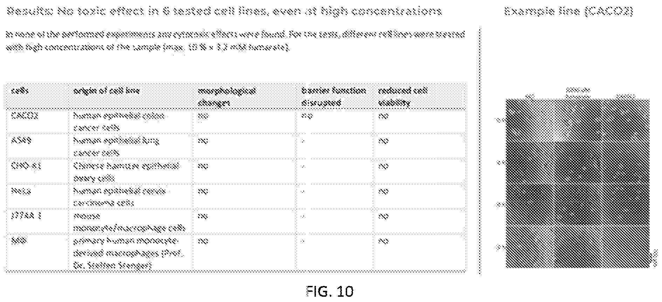

The clean fumarate samples following precipitation were tested for cytotoxicity using a variety of cell lines. **FIG. 10** depicts the result of the cell line experiments. No toxicity was visible even at high concentrations.

Preclinical MRI experiments were conducted using the 1-13C hyperpolarized sodium fumarate after precipitation, washing and redissolution. The experiments were performed in a 11.7T Bruker preclinical MRI. The hyperpolarized fumarate was injected through a tail vein of the mouse and imaged using a ¹³C coil insert. Images and scan parameters are given in

### FIG. 11.

### Exemplary Large-Volume Polarization Transfer

Experiments were performed on the experimental setup of **FIG. 9A****,** with the modification that the magnetic shield was placed vertically in order to have access through the larger axis of the shield for large volumes. The starting solution contains 400 mM acetylenedicarboxylic acid monosodium salt, 250 mM sodium sulphite anhydrous and 8 mM tris(acetonitrile)pentamethylcyclopentadienyl ruthenium(ii) hexafluorophosphate catalyst. This solution is titrated with 1 M NaOD to a pH of 7.8. The solution is then filled in an ETFE coated aluminum reactor and heated to 90 °C. Subsequently the parahydrogen is bubbled through a frit into the solution at a pressure 10 bar and with a flow of 2 liter per minute. The hydrogenation is stopped after 20s. Approximately 2 ml of the hydrogenated sample were then injected into a vial containing 45 mL of D₂O which was placed into the magnetic shield. Inside the magnetic shield a linear magnetic field sweep is carried out. The field sweep starts at 50 nT and ramps up to 1000 nT within 5 seconds, similar to the sweep depicted in **FIG. 9B****.**

The magnetic shield (MS2, Twinleaf LLC, Princeton, U.S.) was used. The magnetic field shimming and sweeps were produced using a home made solenoid piercing the shield top through bottom along the z-axis, powered by the analog output of a national instruments card (PCIe-6363,National Instruments US).

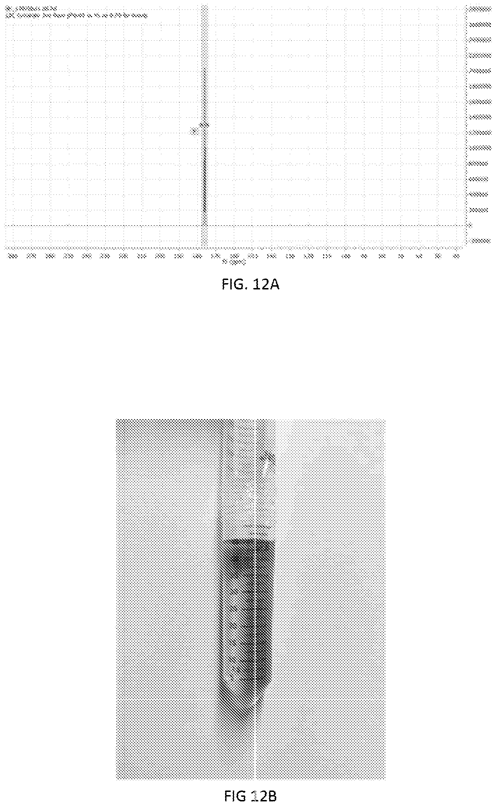

Following the magnetic field sweep, a hyperpolarized sample with a volume of roughly 47 ml was obtained. **FIG. 12B** shows the sample in the vial. For measuring the polarization, a small sample from the vial was measured in an 2T Fourier80 NMR spectrometer. **FIG. 12A** depicts the ¹³C NMR spectrum of hyperpolarized sodium fumarate-1-¹³C (80 MHz, D₂O). The polarization of the ¹³C nuclear spin was calculated to be 36%. The ¹³C polarization was calculated by comparing the hyperpolarized signal intensity with a reference sample (99% labeled methanol) of known ¹³C enrichment and concentrations at thermal equilibrium. Together with the knowledge of the concentration (measured via NMR) and ¹³C enrichment (also measured via NMR or natural abundance) this comparison yields the signal enhancement. When multiplying the thermal polarization of ¹³C at the measurement field of the NMR one obtains the polarization which is referenced in this text.

The foregoing description has been presented for purposes of illustration. It is not exhaustive and is not limited to precise forms or embodiments disclosed. Modifications and adaptations of the embodiments will be apparent from consideration of the specification and practice of the disclosed embodiments. For example, the described implementations include hardware, but systems and methods consistent with the present disclosure can be implemented with hardware and software. In addition, while certain components have been described as being coupled to one another, such components may be integrated with one another or distributed in any suitable fashion.

Embodiments herein include systems, methods, and tangible non-transitory computer-readable media. The methods may be executed, at least in part for example, by at least one processor that receives instructions from a tangible non-transitory computer-readable storage medium. Similarly, systems consistent with the present disclosure may include at least one processor and memory, and the memory may be a tangible non-transitory computer-readable storage medium. As used herein, a tangible non-transitory computer-readable storage medium refers to any type of physical memory on which information or data readable by at least one processor may be stored. Examples include random access memory (RAM), read-only memory (ROM), volatile memory, non-volatile memory, hard drives, CD ROMs, DVDs, flash drives, disks, registers, caches, and any other known physical storage medium. Singular terms, such as "memory" and "computer-readable storage medium," may additionally refer to multiple structures, such a plurality of memories or computer-readable storage media. As referred to herein, a "memory" may comprise any type of computer-readable storage medium unless otherwise specified. A computer-readable storage medium may store instructions for execution by at least one processor, including instructions for causing the processor to perform steps or stages consistent with embodiments herein. Additionally, one or more computer-readable storage media may be utilized in implementing a computer-implemented method. The term "non-transitory computer-readable storage medium" should be understood to include tangible items and exclude carrier waves and transient signals.

Moreover, while illustrative embodiments have been described herein, the scope includes any and all embodiments having equivalent elements, modifications, omissions, combinations (e.g., of aspects across various embodiments), adaptations or alterations based on the present disclosure. The elements in the claims are to be interpreted broadly based on the language employed in the claims and not limited to examples described in the present specification or during the prosecution of the application, which examples are to be construed as nonexclusive. Further, the steps of the disclosed methods can be modified in any manner, including reordering steps or inserting or deleting steps.

The features and advantages of the disclosure are apparent from the detailed specification, and thus, it is intended that the appended claims cover all systems and methods falling within the scope of the invention as defined by the claims. As used herein, the indefinite articles "a" and "an" mean "one or more." Similarly, the use of a plural term does not necessarily denote a plurality unless it is unambiguous in the given context. Further, since numerous modifications and variations will readily occur from studying the present disclosure, it is not desired to limit the disclosure to the exact construction and operation illustrated and described, and accordingly, all suitable modifications and equivalents may be resorted to, falling within the scope of the invention as defined by the appended claims.

Some embodiments may be described by the following clauses:
24. A system for generating a polarized solution comprising a hyperpolarized target compound, the system comprising: a first sub-system for generating a hyperpolarized precipitate, the first sub-system comprising: a hydrogenation device configured to generate a first solution by combining parahydrogen gas, a solvent, and a precursor or a target compound; a polarization device configured to receive the first solution and generate a second solution, the polarization device including a polarization chamber having a volume of at least 1 ml; a waveform generator coupled to the polarization device, the waveform generator configured to provide a polarization transfer signal; and a separation system configured to separate a precipitated fraction of the hyperpolarized target compound from the second solution; and a second sub-system for dissolution of the precipitated fraction, the second sub-system comprising a dissolution chamber configured to contain the precipitated fraction and to receive a second solvent for dissolving the precipitated fraction.
25. The system of clause 24, wherein: the polarization device comprises one or more radiofrequency coils and a magnetic field source, wherein:
   the one or more radiofrequency coils are disposed around the polarization chamber; and
   the magnetic field source is disposed around the polarization chamber; and
   the waveform generator is coupled to the one or more radiofrequency coils, and the polarization transfer signal comprises a polarization waveform.
26. The system of clause 24, wherein: the polarization device comprises magnetic field coils disposed around the polarization chamber and a magnetic shield disposed around the magnetic field coils; and the waveform generator is coupled to the magnetic field coils of the polarization device, and the polarization transfer signal is configured to modulate a magnetic field applied to the polarization chamber.
27. The system of clause 26, wherein: the magnetic shield is configured to maintain a magnetic field strength within the polarization chamber at less than 10 micro Tesla during modulation of the magnetic field in the polarization chamber.
28. The system of any one of clauses 24-27, wherein: the hydrogenation device comprises a bubbler configured to introduce the parahydrogen gas into the solvent; a membrane configured to enable diffusion of the parahydrogen gas into the solvent; or an aerosolizer configured to spray droplets of the solvent into the parahydrogen chamber configured to receive the parahydrogen gas.
29. The system of any one of clauses 24-28, wherein the separation system comprises a precipitation chamber configured to receive the polarized solution, the precipitation chamber including: a reagent port for introducing a precipitation agent into the precipitation chamber; or a stimulation port for introducing an electromagnetic precipitation radiation into the precipitation chamber.
30. The system of any one of clauses 24-29, further comprising a transportation device, the transportation device including a transport chamber configured to receive the precipitated fraction and a cooling system, the transportation device configured to maintain the transport chamber within a predetermined temperature range and a predetermined magnetic field strength.
31. The system of clause 30, wherein the transportation device further includes a magnetic shielding system configured to maintain a magnetic field strength within the transport chamber of at least 10 milli Tesla.
32. The system of any one of clauses 24-31, wherein the volume of the polarization chamber is at least 5 ml.
33. A method, comprising: modulating an amplitude of a magnetic field applied to a sample contained within a magnetic shield, a duration of the modulation being greater than 100 milliseconds and less than 20,000 milliseconds, the amplitude of the magnetic field during modulation being less than 2 microteslas, and a spatial deviation of the magnetic field during modulation being less than 0.5 microteslas over a volume of the sample greater than 10 milliliters and less than 2,000 milliliters; and wherein the modulation is configured to transfer a population difference in parahydrogenated proton spin states to polarization on a target nuclear spin of a parahydrogenated precursor in the sample.
34. The method of clause 33, wherein, following transfer, the parahydrogenated precursor exhibits at least 10% nuclear spin state polarization.
35. The method of any one of clauses 33-34, wherein a maximum of a rate of change of the amplitude of the magnetic field during the modulation is greater than 1 uT per second.
36. The method of any one of clauses 33-34, wherein modulating the amplitude of the magnetic field comprises linearly varying the amplitude of the magnetic field.
37. The method of any one of clauses 33-36, wherein a sign of a rate of change of the amplitude of the magnetic field changes at least three times during the modulation.
38. The method of any one of clauses 33-37, wherein the amplitude of the magnetic field: increases during at least two increasing intervals of the modulation, and decreases during at least two decreasing intervals of the modulation.
39. The method of any one of clauses 33-34, wherein modulating the amplitude of the magnetic field comprises setting the magnetic field to a fixed amplitude.
40. The method of any one of clauses 33-39, wherein the amplitude of the magnetic field is modulated by applying an electrical signal to magnetic field coils within the magnetic shield.
41. The method of any one of clauses 33-40, wherein the volume of the magnetic shield is greater than 30 ml.
42. The method of any one of clauses 33-41, wherein the method further comprises: placing the sample within the magnetic shield; and flowing, through the sample before or during modulation of the amplitude of the magnetic field, parahydrogen having at least 10% population in the parahydrogen spin state.
43. The method of clause 42, wherein the parahydrogen has at least a 40% population in the parahydrogen spin state.
44. The method of any one of clauses 33-43, wherein the method further comprises: generating, during or after modulation of the amplitude of the magnetic field, at least 1 ml of a first mixture including at least 10 mM of a target compound by cleaving the parahydrogenated precursor into the compound and a sidearm and separating the first mixture from the sample.
45. The method of clause 44, wherein the parahydrogenated precursor is cleaved into the biorelevant imaging agent and the side-arm using a hydrolyzing agent.
46. The method of clause 44, wherein the first mixture is separated from the sample using liquid-liquid separation.
47. A system, comprising: a magnetic shield; a sample reservoir disposed within the magnetic shield; a magnetic field coil disposed within the magnetic shield, electrically connected to a signal generator, and configured to generate, when driven by the signal generator, a magnetic field, a spatial deviation of the magnetic field being, when an amplitude of the magnetic field is less than 2 uT, less than 0.5 uT over a volume of the sample reservoir, the volume of the sample reservoir being greater than 10 ml and less than 2,000 ml; and at least one computer-readable media containing instructions that, when executed by at least one processor of the system, cause the system to perform operations comprising: modulating the amplitude of the magnetic field, a duration of the modulation being greater than 100 milliseconds and less than 20,000 milliseconds, the amplitude of the magnetic field during modulation being less than 2uT; and wherein the modulation is configured to transfer a population difference in parahydrogenated proton spin states to polarization on a target nuclear spin of a target compound in the sample contained in the sample reservoir.
48. The system of clause 47, wherein modulating the amplitude of the magnetic field comprises linearly varying the amplitude of the magnetic field.
49. The system of any one of clauses 47-48, wherein a maximum of a rate of change of the amplitude of the magnetic field during the modulation is greater than 1 uT per second.
50. The system of any one of clauses 47-49, wherein a sign of a rate of change of the amplitude of the magnetic field changes at least at three times during the duration of the modulation.
51. The system of any one of clauses 47-50, wherein the amplitude of the magnetic field: increases during at least two increasing intervals of the duration of the modulation, and decreases during at least two decreasing intervals of the duration of the modulation.
52. The system of any one of clauses 47-51, wherein the amplitude of the magnetic field is modulated by applying an electrical signal to magnetic field coils within the magnetic shield.
53. The system of any one of clauses 47-52, wherein the volume of the magnetic shield is greater than 30 ml.
54. The system of any one of clauses 47-53, further comprising a parhydrogenation chamber disposed outside the magnetic shield, the parahydrogenation chamber connected to the sample reservoir.

As used herein, unless specifically stated otherwise, the term "or" encompasses all possible combinations, except where infeasible. For example, if it is stated that a component may include A or B, then, unless specifically stated otherwise or infeasible, the component may include A, or B, or A and B. As a second example, if it is stated that a component may include A, B, or C, then, unless specifically stated otherwise or infeasible, the component may include A, or B, or C, or A and B, or A and C, or B and C, or A and B and C.

## Claims

1. A method of generating a solution comprising a hyperpolarized target compound, the method comprising:
generating a first solution comprising an organic solvent, the first solution having a first concentration of a precursor of a target compound, by inducing a hydrogenation reaction between parahydrogen and the precursor using parahydrogen induced polarization "PHIP", thereby creating a population difference in proton spins in the parahydrogenated precursor;
applying a polarization transferring waveform to the first solution, the polarization transferring waveform configured to transfer the created population difference to polarization on a target nuclear spin of the parahydrogenated precursor;
precipitating a first amount of the target compound from the first solution comprising the organic solvent;
separating the precipitated first amount from the first solution; and
generating a second solution having a second concentration of the target compound by combining the precipitated first amount with a solvent.

2. The method of claim 1, wherein:
precipitating the first amount of the target compound from the first solution comprises:
modifying at least one of a temperature, acidity, or pressure of the first solution.

3. The method of claim 1, wherein:
precipitating the first amount of the target compound from the first solution comprises any one of:
modifying an acidity of the first solution, wherein the acidity of the first solution is modified by adding a base to the first solution;
applying electromagnetic stimulation configured to reduce solubility of the target compound by modifying a structure of the target compound.

4. The method of claim 1, wherein:
the second concentration is greater than the first concentration.

5. The method of claim 1, wherein:
the target compound is pyruvate, glutamate, glutamine, lactate, acetate, acetoacetate, zymonate, or a conjugate acid of any of these compounds.

6. The method of claim 1, wherein:
the transferring of the created population difference comprises any one of:
applying a polarization transferring waveform to the first solution; and
locating the first solution in a magnetically shielded environment.

7. The method of claim 1, wherein:
the method further comprises cleaving the parahydrogenated precursor to generate the target compound; or
the parahydrogenated precursor comprises the target compound.

8. A method of generating a solution comprising a hyperpolarized target compound, the method comprising:
generating a first solution including an organic solvent, a target compound, parahydrogen, and a catalyst, the catalyst configured to bind the parahydrogen and the target compound, optionally wherein the catalyst comprises an iridium organometallic complex;
transferring spin order from the parahydrogen to polarization on the target compound via the catalyst using the SABRE effect;
generating a second solution including the target compound and the catalyst, the generation comprising:
generating a precipitate of the target compound; separating the precipitate from the first solution comprising the organic solvent; and
combining at least some of the precipitate with a solvent, thereby generating the solution comprising the hyperpolarized target compound.

9. The method of claim 8, wherein the transferring spin order to polarization on the target compound comprises applying a polarization transferring waveform to the first solution; and optionally wherein
the polarization transferring waveform comprises a SABRE waveform.

10. The method of claim 8, wherein the transferring spin order to polarization on the target compound comprises locating the first solution in a magnetically shielded environment.

11. The method of claim 8, wherein the generating of the precipitate of the target compound comprises reducing a solubility of the target compound in the first solution, and optionally wherein
reducing of the solubility of the target compound in the first solution comprises changing a pH or temperature of the first solution.

12. The method of claim 8, wherein:
the organic solvent comprises deuterated chloroform, deuterated acetone, deuterated ethanol, or deuterated methanol.

13. The method of claim 8, wherein:
the target compound comprises pyridine and the precipitate comprises a pyridinium salt.

## Patentansprüche

1. Verfahren zur Erzeugung einer Lösung, die eine hyperpolarisierte Zielverbindung enthält, wobei das Verfahren umfasst:
Erzeugen einer ersten Lösung, die ein organisches Lösungsmittel enthält, wobei die erste Lösung eine erste Konzentration eines Vorläufers einer Zielverbindung aufweist, indem eine Hydrierungsreaktion zwischen Parawasserstoff und dem Vorläufer unter Verwendung von Parawasserstoff-induzierter Polarisation "PHIP" induziert wird, wodurch eine Populationsdifferenz in den Protonenspins im parawasserstoffierten Vorläufer erzeugt wird;
Anwenden einer Polarisationsübertragungswellenform auf die erste Lösung, wobei die Polarisationsübertragungswellenform so konfiguriert ist, dass sie den erzeugten Populationsunterschied auf die Polarisation eines Zielkernspins des parahydrogenierten Vorläufers überträgt;
Ausfällen einer ersten Menge der Zielverbindung aus der ersten Lösung, die das organische Lösungsmittel umfasst;
Trennen der ausgefällten ersten Menge von der ersten Lösung; und
Erzeugen einer zweiten Lösung mit einer zweiten Konzentration der Zielverbindung durch Kombinieren der ausgefällten ersten Menge mit einem Lösungsmittel.

2. Verfahren nach Anspruch 1, wobei:
das Ausfällen der ersten Menge der Zielverbindung aus der ersten Lösung umfasst:
das Modifizieren mindestens einer der folgenden Größen: Temperatur, Säuregrad oder Druck der ersten Lösung.

3. Verfahren nach Anspruch 1, wobei:
das Ausfällen der ersten Menge der Zielverbindung aus der ersten Lösung eines der folgenden Verfahren umfasst:
Modifizieren der Azidität der ersten Lösung, wobei die Azidität der ersten Lösung durch Zugabe einer Base zur ersten Lösung modifiziert wird;
Anwenden einer elektromagnetischen Stimulation, die so konfiguriert ist, dass sie die Löslichkeit der Zielverbindung durch Modifizieren einer Struktur der Zielverbindung verringert.

4. Verfahren nach Anspruch 1, wobei:
die zweite Konzentration größer ist als die erste Konzentration.

5. Verfahren nach Anspruch 1, wobei:
die Zielverbindung Pyruvat, Glutamat, Glutamin, Laktat, Acetat, Acetoacetat, Zymonat oder eine konjugierte Säure einer dieser Verbindungen ist.

6. Verfahren nach Anspruch 1, wobei:
das Übertragen der erzeugten Populationsdifferenz eines der folgenden Verfahren umfasst:
Anwenden einer Polarisationsübertragungswellenform auf die erste Lösung; und
Anordnen der ersten Lösung in einer magnetisch abgeschirmten Umgebung.

7. Verfahren nach Anspruch 1, wobei:
das Verfahren ferner das Spalten des parahydrogenierten Vorläufers umfasst, um die Zielverbindung zu erzeugen; oder
der parahydrogenierte Vorläufer die Zielverbindung umfasst.

8. Verfahren zur Erzeugung einer Lösung, die eine hyperpolarisierte Zielverbindung enthält, wobei das Verfahren umfasst:
Erzeugen einer ersten Lösung, die ein organisches Lösungsmittel, eine Zielverbindung, Parawasserstoff und einen Katalysator enthält, wobei der Katalysator so konfiguriert ist, dass er den Parawasserstoff und die Zielverbindung bindet, wobei der Katalysator optional einen organometallischen Iridiumkomplex umfasst;
Übertragen der Spinordnung vom Parawasserstoff auf die Polarisation der Zielverbindung über den Katalysator unter Verwendung des SABRE-Effekts;
Erzeugen einer zweiten Lösung, die die Zielverbindung und den Katalysator enthält, wobei das Erzeugen umfasst:
Erzeugen eines Niederschlags der Zielverbindung; Abtrennen des Niederschlags von der ersten Lösung, die das organische Lösungsmittel enthält; und
Kombinieren zumindest eines Teils des Niederschlags mit einem Lösungsmittel, wodurch die Lösung erzeugt wird, die die hyperpolarisierte Zielverbindung enthält.

9. Verfahren nach Anspruch 8, wobei das Übertragen der Spinordnung auf die Polarisation auf die Zielverbindung das Anwenden einer Polarisationsübertragungswellenform auf die erste Lösung umfasst; und wobei optional die Polarisationsübertragungswellenform eine SABRE-Wellenform umfasst.

10. Verfahren nach Anspruch 8, wobei das Übertragen der Spinordnung auf die Polarisation auf die Zielverbindung das Anordnen der ersten Lösung in einer magnetisch abgeschirmten Umgebung umfasst.

11. Verfahren nach Anspruch 8, wobei das Erzeugen des Niederschlags der Zielverbindung das Verringern der Löslichkeit der Zielverbindung in der ersten Lösung umfasst und wobei optional
das Verringern der Löslichkeit der Zielverbindung in der ersten Lösung das Ändern des pH-Werts oder der Temperatur der ersten Lösung umfasst.

12. Verfahren nach Anspruch 8, wobei:
das organische Lösungsmittel deuteriertes Chloroform, deuteriertes Aceton, deuteriertes Ethanol oder deuteriertes Methanol umfasst.

13. Verfahren nach Anspruch 8, wobei:
die Zielverbindung Pyridin umfasst und der Niederschlag ein Pyridiniumsalz umfasst.

## Revendications

1. Procédé de génération d'une solution comprenant un composé cible hyperpolarisé, le procédé comprenant :
la génération d'une première solution comprenant un solvant organique, la première solution ayant une première concentration d'un précurseur d'un composé cible, en induisant une réaction d'hydrogénation entre le parahydrogène et le précurseur à l'aide d'une polarisation induite par le parahydrogène « PHIP », créant ainsi une différence de population dans les spins de protons dans le précurseur parahydrogéné ;
l'application d'une forme d'onde de transfert de polarisation à la première solution, la forme d'onde de transfert de polarisation étant configurée pour transférer la différence de population créée à la polarisation sur un spin nucléaire cible du précurseur parahydrogéné ;
précipiter une première quantité du composé cible à partir de la première solution comprenant le solvant organique ;
séparer la première quantité précipitée de la première solution ; et
générer une deuxième solution ayant une deuxième concentration du composé cible en combinant la première quantité précipitée avec un solvant.

2. Procédé selon la revendication 1, dans lequel :
la précipitation de la première quantité du composé cible à partir de la première solution comprend :
la modification d'au moins l'un parmi la température, l'acidité ou la pression de la première solution.

3. Procédé selon la revendication 1, dans lequel :
la précipitation de la première quantité du composé cible à partir de la première solution comprend l'une quelconque des opérations suivantes :
la modification de l'acidité de la première solution, dans laquelle l'acidité de la première solution est modifiée par l'ajout d'une base à la première solution ;
l'application d'une stimulation électromagnétique configurée pour réduire la solubilité du composé cible en modifiant la structure du composé cible.

4. Procédé selon la revendication 1, dans lequel :
la deuxième concentration est supérieure à la première concentration.

5. Procédé selon la revendication 1, dans lequel :
le composé cible est le pyruvate, le glutamate, la glutamine, le lactate, l'acétate, l'acétoacétate, le zymonate ou un acide conjugué de l'un quelconque de ces composés.

6. Procédé selon la revendication 1, dans lequel :
le transfert de la différence de population créée comprend l'une quelconque des opérations suivantes :
appliquer une forme d'onde de transfert de polarisation à la première solution ; et
placer la première solution dans un environnement blindé magnétiquement.

7. Procédé selon la revendication 1, dans lequel :
le procédé comprend en outre le clivage du précurseur parahydrogéné pour générer le composé cible ; ou
le précurseur parahydrogéné comprend le composé cible.

8. Procédé de génération d'une solution comprenant un composé cible hyperpolarisé, le procédé comprenant :
la génération d'une première solution comprenant un solvant organique, un composé cible, du parahydrogène et un catalyseur, le catalyseur étant configuré pour lier le parahydrogène et le composé cible, dans lequel, en option, le catalyseur comprend un complexe organométallique d'iridium ;
transférer l'ordre de spin du parahydrogène à la polarisation sur le composé cible via le catalyseur en utilisant l'effet SABRE ;
générer une deuxième solution comprenant le composé cible et le catalyseur, la génération comprenant :
générer un précipité du composé cible ; séparer le précipité de la première solution comprenant le solvant organique ; et
combiner au moins une partie du précipité avec un solvant, générant ainsi la solution comprenant le composé cible hyperpolarisé.

9. Procédé selon la revendication 8, dans lequel le transfert de l'ordre de spin vers la polarisation sur le composé cible comprend l'application d'une forme d'onde de transfert de polarisation à la première solution ; et dans lequel, éventuellement,
la forme d'onde de transfert de polarisation comprend une forme d'onde SABRE.

10. Procédé selon la revendication 8, dans lequel le transfert de l'ordre de spin vers la polarisation sur le composé cible comprend le placement de la première solution dans un environnement blindé magnétiquement.

11. Procédé selon la revendication 8, dans lequel la génération du précipité du composé cible comprend la réduction de la solubilité du composé cible dans la première solution, et dans lequel, éventuellement,
la réduction de la solubilité du composé cible dans la première solution comprend la modification du pH ou de la température de la première solution.

12. Procédé selon la revendication 8, dans lequel :
le solvant organique comprend du chloroforme deutéré, de l'acétone deutérée, de l'éthanol deutéré ou du méthanol deutéré.

13. Procédé selon la revendication 8, dans lequel :
le composé cible comprend de la pyridine et le précipité comprend un sel de pyridinium.
